# EUROPEAN PATENT APPLICATION

(11) **EP 4 782 545 A1**
(43) Date of publication of application: **29.07.2026**
(21) Application number: 24867539.9
(22) Date of filing: 20.09.2024
(51) Int. Cl.: C12N 15/86, A61K 39/215

(54) **PREPARATION, PURIFICATION AND USE OF PROTEIN COMPLEX**

(30) Priority: 22.09.2023 CN 202311236032
(71) Applicant: Beijing Cytoship Biotechnology Co., Ltd., Beijing 100102 (CN)
(72) Inventor: JIANG, Feng, Beijing 100102 (CN); JIN, Qi, Beijing 100102 (CN); WANG, Xia, Beijing 100102 (CN); FENG, Xiao, Beijing 100102 (CN); WANG, Yueying, Beijing 100102 (CN)
(74) Representative: karo IP
(86) International application number: PCT/CN2024/119978
(87) International publication number: WO 2025/061134

(57) **Abstract**

A protein delivery system, and a preparation and use thereof, belonging to the technical field of biology. The protein delivery system comprises a protein complex and a signal peptide, the protein complex comprising a structural protein and a regulatory factor, and the structural protein and the regulatory factor being from *Xenorhabdus khoisanae.* A structural gene and lysR gene of an XkCIS protein complex from the MCB strain of *Xenorhabdus khoisanae* are expressed in *Escherichia coli,* achieving the expression and assembly of the XkCIS protein complex in bacteria. Furthermore, XkCIS is modified, and an effector protein is selectively fused with the signal peptide. Under the guidance of the signal peptide, it is loaded into the XkCIS protein complex, which can achieve targeted delivery of the effector protein.

## Description

### TECHNICAL FIELD

This disclosure relates to the preparation, purification, and use of a protein complex, particularly to a multipurpose protein delivery system, preparation method, and use thereof. The disclosure falls within the field of biotechnology.

### BACKGROUND ART

The secretion system of microorganisms plays an important role in coping with complex living environments. By exporting their own effector molecules, microorganisms stabilize their own physiological processes and maintain the microenvironment in which they live, thereby achieving colonization, internalization and diffusion in host cells. Contractile injection systems (CISs, or Cis), which are widely found in bacteria and archaea, represent an important class of protein secretion systems. They can form a device similar to the contractile tail of a bacteriophage, which transfers bacterial virulence factors to other host cells, mediates intercellular communication, and induces virulence to gain a growth advantage. CISs comprise intracellular CISs and extracellular CISs (eCISs). Intracellular CISs inject virulence factors outwards from cytoplasm in a contact-dependent manner, represented by the type VI secretion system (T6SS) which is widely present in gram-negative bacteria (A. Vettiger, M. Basler, Type VI secretion system substrates are transferred and reused among sister cells. Cell, 2016, 167(1): 99-110; J. Wang, M. Brackmann, D. Castaño-Díez, et al., Cryo-EM structure of the extended type VI secretion system sheath-tube complex. Nat Microbiol, 2017, 2(11): 1507-1512; M. S. Stietz, X. Liang, H. Li, et al., TssA-TssM-TagA interaction modulates type VI secretion system sheath-tube assembly in Vibrio cholerae. Nat Commun, 2020,11(1): 5065), having a similar structure to the contractile tail of T4 phage. After the membrane complex is formed, it anchors to the bacterial cell membrane and penetrates the inner and outer membranes to transport virulence factors to prokaryotic or eukaryotic cells.

Unlike intracellular CISs, eCISs can be released into the extracellular environment and attack host cells from the outside space without requiring direct cell-cell contact. Common eCISs comprise the *Photorhabdus* virulence cassette (PVC) (P. Ge, D. Scholl, P. G. Leiman, et al., atomic structures of abactericidal contractile nanotube in its pre- and postcontraction states. Nat Struct Mol Biol, 2015, 22(5): 377-382), antifeeding prophage (AFP) in *Serratia entomophila* (T. Jank, S. Eckerle, M. Steinemann, et al. Tyrosine glycosylation of Rho by Yersinia toxin impairs blastomere cell behaviour in zebrafish embryos. Nature Communications, 2015, 6 (1): 7807), R-type pyocin secreted by *Pseudomonas aeruginosa* (M. Mei, J. Thomas, S. P. Diggle, Heterogenous susceptibility to R-pyocins in populations of Pseudomonas aeruginosa sourced from cystic fibrosis lungs. mBio, 2021, 12(3): e00458-21), Metamorphosis-associated contractile structure (MAC) secreted by *Pseudoalteromonas luteoviolacea* (N. J. Shikuma, I. Antoshechkin, J. M. Medeiros, et al., Stepwise metamorphosis of the tubeworm Hydroides elegans is mediated by a bacterial inducer and MAPK signaling. Proc Natl Acad Sci U S A, 2016, 113(36):10097-102).

Currently, biological macromolecules, represented by proteins and DNA/RNA, have great potential in the prevention and treatment of major diseases. How to achieve efficient and specific delivery of biological macromolecules is an important scientific and technological problem in this field (J. van Haasteren, J. Li, O. J. Scheideler, et al., The delivery challenge: fulfilling the promise of therapeutic genome editing. Nat Biotechnol 2020, 38(7): 845- 855). In terms of protein delivery technology, physical methods such as electroporation and microinjection require instruments to perforate the cell membrane before delivering proteins into the cell, resulting in low efficiency and limited clinical application. Virus-like protein delivery systems (eVLPs) can be used for efficient in vivo delivery of gene-edited protein RNP complexes, enabling the efficient delivery of therapeutic protein RNP complexes to primary cells and various mouse tissues, and have achieved good therapeutic effects in various disease models (S. Banskota, A. Raguram, S. Suh, et al., Engineered virus-like particles for efficient in vivo delivery of therapeutic proteins. Cell, 2022, 185(2): 250-265). The applicant previously conducted a detailed analysis of the construction and delivery function of eCIS by using PVC as the research object, for example, the preparation and structure of its complete vector (Jiang Feng, et al., 2019. Cell. 177(2): 370-383 e15); loading and action mechanism of PVC effector proteins (Wang xia, et al., 2022. Science China Life Sciences. 65(3): 618-630). The discovery of effector signal peptides can help PVC recognize, load and transport target proteins, thereby mediating the effective delivery of target proteins to mouse cells and the mouse body (F. Jiang, J. Shen, J. Cheng, et al., Sci Adv 2022, 8(17): eabm2343). Recently, Zhang Feng's team from the Broad Institute further modified the PVC vector, taking it a step closer to being used for human cell delivery (Joseph Kreitz, et al., Nature 2023. doi.org/10.1038/s41586-023-05870-7).

Peptide and protein medicaments, with their advantages of high efficacy, low toxicity, and high specificity, are gradually gaining public attention and have broad prospects for clinical application. Compared to small molecule medicaments, protein medicaments are difficult to penetrate cell membranes and enter the cytoplasm. Therefore, it is necessary to develop effective protein delivery technologies to lay a foundation for the widespread application of protein medicaments. Compared to DNA/RNA-based delivery technologies, research on new protein delivery technologies has progressed slowly and still requires new breakthroughs. Although physical methods such as electroporation and microinjection have made many breakthroughs, research on specific delivery still faces a number of challenges. Non-specific delivery can easily lead to safety risks, which greatly limits the application prospects of protein medicaments and the development of targeted therapies for diseases.

### SUMMARY

### The Problem to be Solved

Despite progress in related research, effective systems for delivering protein therapeutics into cells remain limited. How to load effector proteins into a delivery system to form a protein complex, thereby achieving efficient and specific protein delivery, it is also an urgent problem to be solved.

### Solutions for Solving the Problem

[1]. A protein delivery system comprising a protein complex and a signal peptide, wherein,
   the protein complex enables the protein delivery system to target the cells of interest, the signal peptide guides the loading of effector proteins into the protein complex, and the protein delivery system is an extracellular retractable injection system (designated XkCIS or XkCis) derived from *Xenorhabdus khoisanae,* optionally from *Xenorhabdus khoisanae* is an MCB strain.
[2]. The protein delivery system according to item [1], wherein the protein complex comprises a structural protein and a regulatory factor, and the structural protein is encoded by the *Cis* gene cluster of *Xenorhabdus khoisanae;* optionally the structural protein is encoded by the XkCis1-16 *Cis* gene cluster of *Xenorhabdus khoisanae* MCB strain; optionally the targeting recognition region in the *Cis* gene cluster is modified to recognize cells of interest.
[3]. The protein delivery system according to item [2], wherein the targeting recognition region comprises the C-terminal amino acids 50-200 aa, 60-180 aa, 70-160 aa, 80-140 aa, 60-120 aa, 60-100 aa, or 60-80 aa region of the XkCis13 protein; optionally the targeting recognition region comprises the region of amino acids 355-419 in SEQ ID NO: 29; and optionally the targeting recognition region comprises the region of amino acids 360-406 in SEQ ID NO: 29.
[4]. The protein delivery system according to item [2] or [3], wherein the targeting recognition region comprises a nucleotide sequence encoding a receptor, ligand, antibody, or a binding domain fragment thereof that recognizes a surface molecule of the cell of interest.
[5]. The protein delivery system according to any one of items [2]-[4], wherein the XkCis1-16 *Cis* gene cluster comprises a nucleotide sequence consisting of the sequences set forth in SEQ ID NO: 47 and SEQ ID NO: 53 in the order from 5' end to 3' end, or a nucleotide sequence having at least 70%, 75%, 80%, 85%, 90%, 95%, or 99% or higher sequence identity with the nucleotide sequence consisting of the sequences set forth in SEQ ID NO: 47 and SEQ ID NO: 53 in the order from 5' end to 3' end.
[6]. The protein delivery system according to any one of items [2]-[5], wherein the regulatory factor comprises the amino acid sequence set forth in SEQ ID NO: 34, or comprises an amino acid sequence having at least 70%, 75%, 80%, 85%, 86%, 87%, 88%, 89%, 90%, 91%, 92%, 93%, 94%, 95%, 96%, 97%, 98%, or 99% identity with SEQ ID NO: 34.
[7]. The protein delivery system according to any one of items [1]-[6], wherein the signal peptide comprises the amino acid sequence set forth in any one of SEQ ID NOs: 44-46, 54-68, or comprises an amino acid sequence having at least 70%, 75%, 80%, 85%, 86%, 87%, 88%, 89%, 90%, 91%, 92%, 93%, 94%, 95%, 96%, 97%, 98%, or 99% identity with the above amino acid sequence;
   optionally the signal peptide comprises the amino acid sequence set forth in any one of SEQ ID NOs: 60-62;
   optionally the signal peptide comprises one or more of: the amino acid sequence set forth in SEQ ID NO: 38, 39 or 40, or a fragment thereof;
   optionally the fragment is a C-terminal truncated fragment of the amino acid sequence set forth in SEQ ID NO: 38, 39 or 40.
[8]. The protein delivery system according to any one of items [1]-[7], wherein the C-terminus of the signal peptide is connected to the N-terminus of the effector protein; optionally a linker sequence is interposed between the signal peptide and the effector protein.
[9]. The protein delivery system according to any one of items [1]-[8], wherein the effector protein comprises any target protein to be administered to a subject or cells of interest;
   optionally the effector protein comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cellular metabolism, gene-editing protein, antigen or immunogen, and/or pharmaceutically active protein.
[10]. An isolated polynucleotide encoding the protein delivery system according to any one of items [1]-[9].
[11]. The polynucleotide according to item [10], wherein the polynucleotide comprises any one or more selected from the group consisting of:
   i) nucleotides encoding a structural protein in the protein delivery system;
   ii) nucleotides encoding a regulatory factor in the protein delivery system; and
   iii) nucleotides encoding a signal peptide in the protein delivery system; and
   optionally nucleotides encoding an effector protein.
[12]. An expression vector comprising the polynucleotide according to item [10] or [11].
[13]. A recombinant host cell comprising the protein delivery system according to any one of items [1]-[9], the polynucleotide according to item [10] or [11], or the expression vector according to item [12].
[14]. A method for preparing the protein delivery system according to any one of items [1]-[9], comprising culturing the host cell according to item [13] to prepare the protein delivery system according to any one of items [1]-[9].
[15]. Use of the protein delivery system of any one of items [1]-[9], the polynucleotide of item [10] or [11], the expression vector of item [12], or the recombinant host cell of item [13] in the preparation of a medicament, reagent, and/or kit for delivering a biological macromolecule.
[16]. The use according to item [15], wherein the biological macromolecule comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cell metabolism, gene-editing protein, antigen or immunogen, and/or pharmaceutically active protein.
[17]. A pharmaceutical composition comprising: the protein delivery system according to any one of items [1]-[9], and
   optionally a pharmaceutically acceptable carrier or excipient;
   optionally the effector protein comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cellular metabolism, gene-editing protein, antigen or immunogen, and/or pharmaceutically active protein.
[18]. A kit for delivering an effector protein, comprising: the protein delivery system according to any one of items [1]-[9], or the pharmaceutical composition according to item [17];
   wherein the effector protein comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cellular metabolism, gene-editing protein, antigen or immunogen, and/or pharmaceutically active protein.

### The effects of the Application

This disclosure achieves the expression and assembly of the XkCIS protein complex in bacteria through co-expression of its structural genes and the lysR gene. Furthermore, the effector protein is selectively fused with a signal peptide and loaded into the XkCIS protein complex under the guidance of the signal peptide, thereby achieving specific delivery of the effector protein.

### BRIEF DESCRIPTION OF THE DRAWINGS

Fig. 1 is a schematic diagram showing the construction of recombinant plasmid pRK404-XkCIS.
Fig. 2 is a schematic diagram showing the construction of recombinant plasmid pBR322-Xk-LysR.
Fig. 3 shows the negative staining electron microscopy image of the XkCIS protein complex.
Fig. 4 shows the SDS-PAGE analysis results of the XkCIS protein complex.
Fig. 5 is a schematic diagram showing the locations of the XkCIS gene cluster and downstream effector factors.
Fig. 6 shows that the Xk1, 2, and 3 signal peptides can guide the loading of TcsT protein into the XkCIS protein complex.
Fig. 7 shows the loading of TcsT protein into the XkCIS protein complex guided by Xk1 signal peptides of varying lengths.
Fig. 8 shows the loading of TcsT protein into the XkCIS protein complex guided by Xk2 signal peptides of varying lengths.
Fig. 9 shows the loading of TcsT protein into the XkCIS protein complex guided by Xk3 signal peptides of varying lengths.
Fig. 10 shows how different signal peptides guide the loading of different proteins into the XkCIS protein complex.
Fig. 11 is a schematic diagram showing the modification of the XkCis13 recognition region.
Fig. 12 shows the viability of Y79 cells following a 36-hour treatment with the modified XkCIS protein complex.
Fig. 13 shows the transmission electron microscopy image of the modified XkCIS particles.

### DETAILED DESCRIPTION

The various exemplary examples, features, and aspects of this disclosure will be described in detail below. The term "exemplary" as used herein means "used as an example, embodiment or illustration". Any example illustrated herein as "exemplary" should not be construed as superior to or better than other examples.

In addition, numerous particular details are given in the following detailed embodiments to better illustrate this disclosure. Those skilled in the art will understand that this disclosure can be practiced even without certain particular details. In other examples, methods, means, equipment and steps well known to those skilled in the art have not been described in detail in order to highlight the spirit of this disclosure.

Unless otherwise stated, all units used herein are international standard units, and all numerical values and ranges appearing in this disclosure should be understood to comprise systematic errors that are unavoidable in industrial production.

As used herein, the word "may" has two meanings: to perform a certain process and not to perform a certain process.

As used herein, the terms "some particular/preferred embodiments", "other particular/preferred embodiments", "embodiment" etc., refer to particular elements (e.g., features, structures, properties and/or characteristics) related to the described embodiment that are comprised in at least one of the embodiments described herein, and may exist in other embodiments or may not exist in other embodiments. Furthermore, it should be understood that the elements can be combined in any suitable manner in various embodiments.

As used herein, the range of values referred to as "value A to value B" refers to the range including the endpoint values A and B.

As used herein, when "normal temperature" or "room temperature" is used, the temperature can be 10-40°C.

As used herein, the terms "polypeptide," "enzyme," "polypeptide or enzyme," or "polypeptide/enzyme" have the same meaning, and are interchangeable in this disclosure. The above terms refer to a polymer composed of many amino acids via peptide bonds, which may or may not contain modifications such as phosphate and formyl groups.

As used herein, the term "amino acid" can include natural amino acids, non-natural amino acids, amino acid analogs, and all their D and L stereoisomers. The amino acids and their abbreviations and English abbreviations in this disclosure are as follows:
histidine (His, H); serine (S); glutamic acid (Glu, E); glutamine (Gln, Q); glycine (Gly, G); threonine (Thr, T); phenylalanine (Phe, F); aspartic acid (Asp, D); tyrosine (Tyr, Y); leucine (Leu, L); isoleucine (Ile, I); arginine (Arg, R); alanine (Ala, A); valine (Val, V); tryptophan (Trp, W); methionine (Met, M); asparagine (Asn, N); cysteine (Cys, C); lysine (Lys, K); and proline (Pro, P).

As used herein, the term "fragment" means a polypeptide or a catalytic or carbohydrate-binding module that has one or more (e.g., several) amino acids missing from the amino and/or carboxyl terminus of a mature polypeptide or domain.

As used herein, the terms "sequence identity" or "identity percentage" in comparisons of two nucleic acids or peptides refer to a particular percentage number of identical nucleotides or amino acids or having the same sequence when compared and aligned with the highest possible correspondence by using nucleotide or amino acid residue sequence comparison algorithms or by visual inspection. In other words, the identity of a nucleotide or amino acid sequence can be defined by the following ratio, which is the proportion of the number of identical nucleotides or amino acids in the total number of nucleotides or amino acids in the aligned portion when comparing two or more nucleotide or amino acid sequences according to the method of maximizing the number of identical nucleotides or amino acids, and adding gaps as needed for alignment.

The methods disclosed herein for determining "sequence identity" or "percentage of identity" include, but are not limited to: those of Computational Molecular Biology, edited by Lesk, A.M., Oxford University Press, New York, 1988; Biocomputing: Informatics and Genome Projects, edited by Smith, D.W., Academic Press, New York, 1993; Computer Analysis of Sequence Data, Part 1, edited by Griffin, A.M. and Griffin, H.G., Humana Press, New Jersey, 1994; Sequence Analysis in Molecular Biology, von Heinje, G., Academic Press, 1987; and Sequence Analysis Primer, edited by Gribskov, M. and Devereux, J. M., Stockton Press, New York, 1991, and Carillo, H. and Lipman, D., SIAM J. Applied Math., 48:1073 (1988). The preferred method for determining identity is to obtain the greatest match between the tested sequences. The method for determining similarity is compiled into a publicly available computer program. Preferred computer program methods for determining the identity between two sequences include, but are not limited to: the GCG program package (Devereux, J. et al., 1984), BLASTP, BLASTN and FASTA (Altschul, S, F, et al., 1990). The BLASTX program (BLAST Manual, Altschul, S. et al., NCBINLM NIH Bethesda, MD 20894; Altschul, S., et al., 1990) is available to the public from NCBI and other sources. The well-known Smith Waterman algorithm can also be used to determine similarity.

In this disclosure, the terms "nucleic acid molecule," "multinucleotide," "polynucleotide," and "nucleic acid" are used interchangeably to refer to a polymeric form of nucleotides of any length, whether deoxyribonucleotides or ribonucleotides, or the like. Polynucleotides can have any three-dimensional structure, and can perform any known or unknown function. Non-limiting examples of polynucleotides include: gene, gene fragment, exon, intron, messenger RNA (mRNA), transfer RNA, ribosomal RNA, ribozyme, cDNA, recombinant polynucleotide, branched polynucleotide, plasmid, vector, isolated DNA of any sequence, control region, isolated RNA of any sequence, nucleic acid probe, and primer. Nucleic acid molecules can be linear or circular.

In this disclosure, the term "codon optimization" refers to the configuration of a nucleotide sequence encoding a polypeptide to contain codons preferred by the host cell or organism to improve gene expression and translation efficiency in the host cell or organism.

As used herein, the term "isolated" means a substance in a form or environment that does not exist in nature. Non-limiting examples of isolated substances include: (1) any substance that is not naturally occurring; (2) any substance, including but not limited to any enzyme, mutant, nucleic acid, protein, peptide or cofactor, which is at least partially removed from one or more of the naturally occurring components associated with it; (3) any substance that is artificially modified relative to a naturally found substance; or (4) any substance modified by increasing the amount of the substance relative to other components naturally associated with it (e.g., recombinant generation in a host cell; multiple copies of the gene encoding the substance; and the use of a promoter stronger than the promoter naturally associated with the gene encoding the substance). The isolated substance can be present in the fermentation broth sample. For example, host cells can be genetically modified to express the polypeptides disclosed herein. Fermentation broth from the host cell will contain isolated peptides. "Recombinant polynucleotides" are a type of "polynucleotide".

As used herein, the term "expression" includes any step involved in polypeptide production, including but not limited to: transcription, post-transcriptional modification, translation, post-translational modification, and secretion.

As used herein, the term "expression vector" refers to a linear or circular DNA molecule containing a polynucleotide encoding a polypeptide, and the polynucleotide is effectively linked to a control sequence for its expression.

As used herein, the term "recombinant expression vector" refers to a DNA structure used to express, for example, a polynucleotide encoding a desired polypeptide. Recombinant expression vectors may include, for example, i) a collection of genetic elements that regulate gene expression, such as promoter and enhancer; ii) a structural or coding sequence to be transcribed into mRNA and translated into a protein; and iii) transcriptional subunits of appropriate transcription and translation initiation and termination sequences. The recombinant expression vector can be constructed in any suitable manner. The nature of the vector is not important, and any vector can be used, including plasmid, virus, bacteriophage, and transposon. Possible vectors used herein include, but are not limited to: chromosomal, non-chromosomal, and synthetic DNA sequences, such as bacterial plasmid, bacteriophage DNA, yeast plasmid, and a vector derived from combination of plasmid and bacteriophage DNA, and DNA from viruses such as vaccinia, adenovirus, fowlpox, baculovirus, SV40, and pseudorabies virus.

As used herein, the terms "transformation," "transfection," and "transduction" have the meaning commonly understood by those skilled in the art as the process of introducing exogenous DNA into a host. The methods of transformation, transfection, and transduction include any method of introducing nucleic acids into cells, including but not limited to: electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method.

As used herein, the term "host cell" means any cell type that is readily transformed, transfected, transduced, etc., by a mutant polypeptide including the mutant polypeptide of this disclosure, a polynucleotide encoding a mutant polypeptide, or a recombinant expression vector.

As used herein, the term "recombinant host cell" encompasses a host cell that differs from the parent cell after the introduction of a polynucleotide or recombinant expression vector encoding a mutant polypeptide, particularly achieved through transformation. The host cell disclosed herein can be a prokaryotic cell or a eukaryotic cell. In one embodiment, the host cell refers to a prokaryotic cell. Particularly, the host cell is derived from microorganisms suitable for fermentation to produce proteins, such as those derived from the *Escherichia, Erwinia, Serratia, Providencia, Enterobacteria, Salmonella, Streptomyces, Pseudomonas, Brevibacterium, Bacillus,* or *Corynebacterium.*

As used herein, the term "linker" can refer to a covalent linker (e.g., a covalent bond), a non-covalent linker, a chemical group, or a molecule that connects two molecules or parts (e.g., two components of a protein complex), for example, two domains of a fusion protein, such as a signal peptide and an effector protein. A linker can be located between or on both sides of two groups, molecules, or other parts, and is connected to each of them by covalent bonds or non-covalent interactions. In some embodiments, the linker may be a polynucleotide. In some embodiments, the linker may be a DNA linker. In some embodiments, the linker may be an RNA linker. In some embodiments, the linker can be one or more amino acids (e.g., a peptide or a protein). In some embodiments, the length of the linker may be about 5-100 amino acids, for example, about 5, 6, 7, 8, 9, 10, 11, 12, 13, 14, 15, 16, 17, 18, 19, 20, 20-30, 30-40, 40-50, 50-60, 60-70, 70-80, 80-90, or 90-100 amino acids. In some embodiments, the length of the linker may be about 100-150, 150-200, 200-250, 250-300, 300-350, 350-400, 400-450, or 450-500 amino acids. Longer or shorter linkers may also be contemplated.

The host cell culture disclosed herein can be performed according to conventional methods in the art, including but not limited to: plate culture, shake flask culture, batch culture, continuous culture, and fed-batch culture, and various culture conditions such as temperature, time, and pH of the culture medium can be appropriately adjusted according to actual conditions.

As used herein, "pharmaceutical composition" means it contains one or more protein delivery systems described herein (e.g., loaded with effector proteins), as well as other components such as physiological/pharmaceutical carriers and excipients. The purpose of a pharmaceutical composition is to facilitate administration to a living organism, thereby promoting the absorption of the active ingredient and enabling it to exert its biological activity.

As used herein, the term "pharmaceutical acceptable" (or "pharmacologically acceptable") means a molecular entity or composition that does not produce an adverse reaction, allergic reaction or other adverse reaction when appropriately administered to an animal or human. As used herein, the term "pharmaceutically acceptable carrier" comprises any and all solvents, dispersion media, coatings, antimicrobial agents, isotonic agents and absorption delay agents, buffers, excipients, adhesives, lubricants, gels, surfactants, etc., that can be used as media for pharmaceutically acceptable substances.

As used herein, the terms "individual" and "subject" are used interchangeably, and refer to mammals. Mammals include, but are not limited to: domesticated animals (e.g., cows, sheep, cats, dogs, and horses), primates (e.g., human and non-human primates such as monkeys), rabbits, and rodents (e.g., mice and rats). In particular, the individual is a person.

The terms "treatment" and "treating" refer to clinical interventions, as described herein, aimed at reversing or alleviating a disease or condition or one or more of its symptoms, delaying the onset of a disease or condition or one or more of its symptoms, or inhibiting the progression of a disease or condition or one or more of its symptoms. As used herein, the terms "treatment" and "treating" refer to clinical interventions as described herein that aim to reverse or alleviate a disease or condition or one or more symptoms thereof, delay the onset of a disease or condition or one or more symptoms thereof, or inhibit the progression of a disease or condition or one or more symptoms thereof. In some embodiments, treatment may be administered after one or more symptoms have developed and/or after the disease has been diagnosed. In other embodiments, treatment may be administered in the absence of symptoms, for example, to prevent or delay the onset of symptoms or to suppress the onset or progression of the disease. For example, treatment can be administered to susceptible individuals before the onset of symptoms (e.g., given the history of symptoms and/or given genetic or other susceptibility factors). Treatment can also continue after symptoms subside, for example, to prevent or delay recurrence.

The technical solutions of this disclosure are described in detail below:

### <Protein Delivery System>

This disclosure provides a protein delivery system comprising a protein complex and a signal peptide, wherein
the protein complex enables the protein delivery system to target the cells of interest, the signal peptide guides the loading of effector proteins into the protein complex, and the protein delivery system is an extracellular retractable injection system from *Xenorhabdus khoisanae* (short for Xk), optionally *Xenorhabdus khoisanae* is an MCB strain.

In some embodiments, the signal peptide is derived from the *Cis (XkCis)* gene cluster of *Xenorhabdus khoisanae,* optionally from *Xenorhabdus khoisanae* MCB strain. In some embodiments, the signal peptide comprises the amino acid sequence set forth in any one of SEQ ID NOs: 44-46, and 54-68, or comprises an amino acid sequence having at least 70%, 75%, 80%, 85%, 86%, 87%, 88%, 89%, 90%, 91%, 92%, 93%, 94%, 95%, 96%, 97%, 98%, or 99% identity with the above sequence,
optionally the signal peptide comprises the amino acid sequence set forth in any one of SEQ ID NOs: 60-62;
optionally the signal peptide comprises one or more of: the amino acid sequence set forth in SEQ ID NO: 38, 39 or 40, or a fragment thereof;
optionally the fragment is a C-terminal truncated fragment of the amino acid sequence set forth in SEQ ID NO: 38, 39 or 40.

In some embodiments, the C-terminus of the signal peptide is directly connected (e.g., via a covalent bond) to the N-terminus of the effector protein; optionally a linker sequence is located between the signal peptide and the effector protein. In some embodiments, the C-terminus of the effector protein may comprise a tag sequence, such as His, FLAG, GST, or other tag sequences.

In some particular embodiments, the effector protein can be any protein, such as any target protein to be administered to a subject/cell, for example, including but not limited to: a virulence factor or reporter protein of *Xenorhabdus khoisanae* MCB strain, a cellular or bacterial virulence protein, transcription factor, regulatory protein (e.g., a signaling pathway regulatory protein), hormone or hormone-regulating molecule, structural protein, transport protein, antimicrobial peptide, enzyme involved in cell metabolism, gene-editing protein, antigen or immunogen, pharmaceutically active protein, and other polypeptides or proteins.

This disclosure reveals that a signal peptide comprising the above sequence is capable of efficiently loading the effector protein into a protein complex.

### Structural Protein

In some embodiments, the protein complex comprises a structural protein. In some embodiments, the structural protein is derived from the *Cis (XkCis)* gene cluster of *Xenorhabdus khoisanae,* optionally from *Xenorhabdus khoisanae* MCB strain. In some embodiments, the structural protein is encoded by the *Cis (XkCis)* gene cluster of *Xenorhabdus khoisanae,* optionally by the XkCis1-16 *Cis* gene cluster of *Xenorhabdus khoisanae* MCB strain, and optionally the targeting recognition region within the *Cis* gene cluster is modified to recognize cells of interest.

In some embodiments, the targeting recognition region is derived from the *XkCis13* gene region of the MCB strain. In some embodiments, the targeting recognition region comprises the C-terminal amino acids 50-200 aa, 60-180 aa, 70-160 aa, 80-140 aa, 60-120 aa, 60-100 aa, or 60-80 aa region of the XkCis13 protein. Optionally the targeting recognition region comprises the region of amino acids 355-419 in SEQ ID NO: 29. Optionally, the targeting recognition region comprises the region of amino acids 360-406 in SEQ ID NO: 29.

In some embodiments, the targeting recognition region is modified to comprise a nucleotide sequence encoding a receptor, ligand, antibody, or a fragment of its binding domain that recognizes a surface molecule of the cell of interest.

In some embodiments, the XkCis1-16 *Cis* gene cluster comprises a nucleotide sequence consisting of the sequences set forth in SEQ ID NO: 47 and SEQ ID NO: 53 in the order from 5' end to 3' end, or a nucleotide sequence having at least 70%, 75%, 80%, 85%, 90%, 95%, or 99% or higher sequence identity with the nucleotide sequence consisting of the sequences set forth in SEQ ID NO: 47 and SEQ ID NO: 53 in the order from 5' end to 3' end.

### Regulatory Factor

In some embodiments, the protein complex further comprises a regulatory factor. In some embodiments, the regulatory factor is derived from the *Cis (XkCis)* gene cluster of *Xenorhabdus khoisanae,* optionally from *Xenorhabdus khoisanae* MCB strain. In some embodiments, the regulatory factor comprises the amino acid sequence set forth in SEQ ID NO: 34, or comprises an amino acid sequence having at least 70%, 75%, 80%, 85%, 86%, 87%, 88%, 89%, 90%, 91%, 92%, 93%, 94%, 95%, 96%, 97%, 98%, or 99% identity with SEQ ID NO: 34.

In some particular embodiments, the regulatory factor comprises a protein expressed by the *lysR* gene of the *Xenorhabdus khoisanae* MCB strain, also referred to herein as Xk-LysR.

### <Polynucleotide>

This disclosure provides an isolated polynucleotide encoding the above protein delivery system.

In some embodiments, the polynucleotide comprises any one or more selected from the group consisting of:
i) nucleotides encoding a structural protein in the above protein delivery system;
ii) nucleotides encoding a regulatory factor in the above protein delivery system; and
iii) nucleotides encoding a signal peptide in the above protein delivery system; and
optionally nucleotides encoding an effector protein.

In some embodiments, the nucleotide sequence encoding the protein delivery system is codon-optimized. This type of optimization may require a mutation in the nucleotide sequence encoding the protein delivery system to mimic the codon preferences of the intended host organism or cell while encoding the same protein.

### <Expression Vector>

This disclosure provides an expression vector comprising the above polynucleotide.

In some particular embodiments, the vector comprises any one or more selected from the group consisting of:
i) nucleotides encoding a structural protein in the above protein delivery system;
ii) nucleotides encoding a regulatory factor in the above protein delivery system; and
iii) nucleotides encoding a signal peptide in the above protein delivery system; and optionally
iv) nucleotides encoding an effector protein.

In some embodiments, any two or more polynucleotide molecules in (i) to (iv) may be present in the same expression vector. In some embodiments, any two or more polynucleotide molecules in (i) to (iv) may be present individually in different expression vectors.

### <Recombinant Host Cell>

Based on the protein delivery system provided in this disclosure, this disclosure provides a recombinant host cell comprising the above protein delivery system, polynucleotide, and expression vector.

In some embodiments, the host cell may include a bacterial, microbial, plant, or animal cell. Bacteria that are easily transformed include members of the *Enterobacteriaceae,* such as a strain of *Escherichia coli* or *Salmonella;* and members of the *Bacillaceae,* such as *Bacillus subtilis.* Suitable microorganisms comprise *Saccharomyces cerevisiae* and *Pichiapastoris.* In some preferred embodiments, the host cell is derived from *Escherichia,* more preferably *Escherichia coli,* including *Escherichia coli* DH10B, *Escherichia coli* Top10, *Escherichia coli* Trans T1, *Escherichia coli* BL21(DE3), etc.

### <Method for Preparing a Protein Delivery System>

This disclosure provides a method for preparing a protein delivery system, which comprises culturing the above host cells to prepare the protein delivery system.

In some particular embodiments, the method may optionally comprise one or more of the following steps: constructing recombinant plasmids expressing structural proteins, expressing regulatory factors and effector proteins for fusion expression of signal peptides, respectively; co-transforming the recombinant plasmids into host cells to construct the above recombinant host cells; and culturing the above recombinant host cells.

In some particular embodiments, a gene cluster encoding a structural protein is constructed into expression vector A to obtain a recombinant plasmid A;
the gene encoding the structural protein is derived from *Xenorhabdus khoisanae* MCB strain; further, the gene encoding the structural protein comprises a polynucleotide sequence consisting of the sequences set forth in SEQ ID NO: 47 and SEQ ID NO: 53 in the order from the 5' end to the 3' end. In other embodiments, the gene encoding the structural protein comprises a polynucleotide sequence as set forth in SEQ ID NOs: 1-16, and the structural protein comprises an amino acid sequence as set forth in SEQ ID NOs: 17-32, or a variant thereof having at least 70%, 75%, 80%, 85%, 90%, 95%, or 99% or higher sequence identity with any one or more of SEQ ID NOs: 17-32. In other embodiments, the structural protein is obtained by modifying the targeting recognition region of a protein encoded by the *Cis* gene cluster into other recognition protein. The other recognition protein can vary depending on the cell of interest to be targeted. For example, the other recognition protein can be a receptor, ligand, antibody, or its binding domain fragment for the surface molecules of the cell of interest to be targeted. For example, it can be a receptor binding fragment, ligand binding fragment, or ScFv fragment, Fv fragment, Fab' fragment or (Fab)2 etc. of an antibody of a cell surface molecule.

Optionally the expression vector A can be any expression vector known in the art, such as pBR322, pRK404, pUC, pBluescript, pBBR1MCS-5 or a modified vector thereof.

In some particular embodiments, a gene encoding a regulatory factor is constructed into expression vector B to obtain recombinant plasmid B; the gene encoding the regulatory factor is derived from *Xenorhabdus khoisanae* MCB strain; the gene encoding the regulatory factor comprises a polynucleotide sequence as set forth in SEQ ID NO: 33, and the regulatory factor comprises an amino acid sequence as set forth in SEQ ID NO: 34, or a variant thereof having at least 70%, 75%, 80%, 85%, 90%, 95%, or 99% or higher sequence identity with SEQ ID NO: 34. Optionally the expression vector B can be any expression vector known in the art, such as pBR322, pRK404, pUC, pACYC184, pBBR1MCS-5 or a modified vector thereof.

In some other particular embodiments, the nucleotides encoding the signal peptide is operatively linked to the gene encoding the effector protein, and then ligated to the expression vector C to construct the recombinant plasmid C. The signal peptide is selected from effector proteins or a fragment thereof from the *Xenorhabdus khoisanae* MCB strain, and the effector protein comprises a sequence set forth in SEQ ID NO: 38, 39 or 40. In some particular embodiments, the fragment may be a C-terminal truncated fragment. In some particular embodiments, the C-terminus can be shortened to any length. In some exemplary embodiments, the C-terminal truncated fragment may be one or more of the 1-10, 1-20, 1-30, 1-40, 1-50, 1-60, 1-70, 1-80, 1-90, 1-100, 1-110, 1-120, or 1-130 amino acids of the amino acid sequence set forth in SEQ ID NO: 38, 39, or 40. As an example, the signal peptide comprises 1-70 or 1-130 amino acids of an amino acid sequence set forth in SEQ ID NO: 38, 39 or 40. For example, in the case of comprising 1-130 amino acids of an amino acid sequence set forth in SEQ ID NO: 38, 39 or 40, the signal peptide may comprise a sequence set forth in SEQ ID NO: 44, 45 or 46. Optionally the expression vector C can be any expression vector known in the art, such as pBad, pRK404, pVTRA, pBluescript, pBBR1MCS-5 or a modified vector thereof, preferably the pBBR1MCS-5 vector or a modified vector thereof. More preferably, the restriction site NdeI is introduced into the LacZα gene of the pBBRIMCS-5 vector to generate a new start codon at the start position of the LacZα gene, thereby constructing the expression vector pBBRN (pBBRN plasmid is disclosed in the literature: Jiang et al., N-terminal signal peptides facilitate the engineering of PVC complex as a potent protein delivery system. Sci Adv. 2022 Apr 29; 8(17):eabm2343, which is incorporated herein by reference) obtained by modifying the pBBR1MCS-5 as a starting vector, and is used for the fusion expression of the signal peptides and effector proteins. In some particular embodiments, the effector protein is one or more selected from the group consisting of: a virulence factor or reporter protein derived from the *Xenorhabdus khoisanae* MCB strain itself, a cellular or bacterial virulence protein, transcription factor, regulatory protein (e.g., signaling pathway regulatory protein), hormone or hormone regulatory molecule, structural protein, transport protein, antimicrobial peptide, enzyme involved in cell metabolism, gene editing protein, antigen or immunogen, and medicament-active proteins. In some exemplary embodiments, the effector protein is TcsT protein, BlaM, Cre, and RluC, etc. Particularly, the TcsT protein is encoded by a nucleotide sequence set forth in SEQ ID NO: 48.

In some alternative embodiments, a step of isolating the proteins from the cultured recombinant host cells is further included. In some alternative embodiments, the steps of purifying the isolated protein and removing endotoxins are further included.

### <Pharmaceutical Composition, Kit, Use and Method>

According to some embodiments of this disclosure, provided is a pharmaceutical composition which comprises the above protein delivery system, and optionally further comprises a pharmaceutically acceptable carrier or excipient.

The term "pharmaceutical acceptable" means that when the molecular itself and the composition are properly administered to animals or humans, they do not produce adverse, allergic, or other adverse reactions.

Examples of substances that can serve as pharmaceutically acceptable carriers or components thereof include: sugars such as lactose, glucose, and sucrose; starches such as corn starch and potato starch; cellulose and its derivatives such as sodium carboxymethyl cellulose, ethyl cellulose, and methyl cellulose; tragacanth gum powder; malt; gelatin; talc; solid lubricants such as stearic acid and magnesium stearate; calcium sulfate; vegetable oils such as peanut oil, cottonseed oil, sesame oil, olive oil, corn oil, and cocoa butter; polyols such as propylene glycol, glycerin, sorbitol, mannitol, and polyethylene glycol; alginic acid; emulsifiers, for example wetting agents such as sodium lauryl sulfate; colorants; flavoring agents; tableting agents; stabilizers; antioxidants; preservatives; pyrogen-free water; isotonic salt solutions and phosphate buffers, etc.

In some embodiments, the compositions disclosed herein can be formulated into various dosage forms as needed, and can be administered by a physician based on factors such as patient type, age, body weight and general disease condition, and route of administration to determine the dosage that is beneficial to the patient. Administration can be done via injection or other treatment methods.

According to some embodiments of this disclosure, provided is a kit for delivering effector proteins, which comprises the above protein delivery system, or the above pharmaceutical composition.

In some particular embodiments, the effector protein is one or more selected from the group consisting of: a virulence factor or reporter protein derived from the *Xenorhabdus khoisanae* MCB strain itself, a cellular or bacterial virulence protein, transcription factor, regulatory protein (e.g., a signaling pathway regulatory protein), hormone or hormone regulatory molecule, structural protein, transport protein, antimicrobial peptide, enzyme involved in cell metabolism, gene-editing protein, antigen or immunogen, and pharmaceutically active protein. In some exemplary embodiments, the effector protein is TcsT protein, BlaM, Cre, and RluC, etc. Particularly, the TcsT protein is encoded by a nucleotide sequence set forth in SEQ ID NO: 48.

According to some embodiments of this disclosure, provided is use of the above protein delivery system in the preparation of a medicament, reagent, and/or kit for delivering a biological macromolecule. According to some embodiments of this disclosure, provided is use of the above protein delivery system for delivering a biological macromolecule. According to some embodiments of this disclosure, this disclosure also provides a method for delivering a biological macromolecule to a subject/cell, which comprises administering the above protein delivery system loaded with the biological macromolecule to the subject/cell. According to some embodiments of this disclosure, this disclosure also provides a method for preventing and/or treating a disease, which comprises administering the above protein delivery system to a subject, wherein the protein delivery system is loaded with a therapeutically effective amount of the biological macromolecule. The disease can be determined based on the function of the biological macromolecule used.

In some particular embodiments, the biological macromolecule comprises one or more selected from the group consisting of: a virulence factor or reporter protein derived from the *Xenorhabdus khoisanae* MCB strain itself, a cellular or bacterial virulence protein, transcription factor, regulatory protein (e.g., a signaling pathway regulatory protein), hormone or hormone regulatory molecule, structural protein, transport protein, antimicrobial peptide, enzyme involved in cell metabolism, gene-editing protein, antigen or immunogen, and pharmaceutically active protein.

In some embodiments of this specification, biological macromolecule, effector protein, and target protein have the same meaning.

### EXAMPLES

The embodiments of the present disclosure are described in detail below with reference to examples. However, it will understand by those skilled in the art that the following examples are provided for illustratve purposes only and are not intended to limit the scope of the present disclosure. Unless otherwise specified, the conditions in the examples conform to the standard conditions or follow the manufacturer's recommendations. If the manufacturers of the reagents or instruments used are not specified, they are all commercially available conventional products.

### Example 1: Construction, Assembly and Purification of XkCIS Complex

### I. Construction of Bacterial Expression System

### 1. Construction of Recombinant Plasmid for Structural Protein

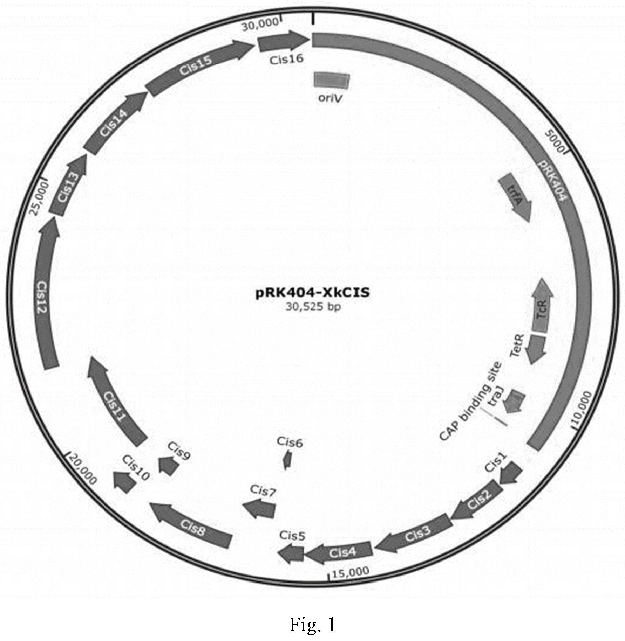

The structural protein gene *XkCIS* is derived from the *Xenorhabdus khoisanae* MCB strain and consists of 16 genes, namely *XkCisl, XkCis2, XkCis3, XkCis4, XkCis5, XkCis6, XkCis7, XkCis8, XkCis9, XkCis10, XkCis11, XkCis12, XkCis13, XkCis14, XkCisl5,* and *XkCisl6.* The gene base sequences and corresponding expressed protein sequences are shown in Table 1. The complete sequence of the Cis gene cluster is shown as the nucleotide sequence composed of the sequences set forth in SEQ ID NO: 47 and SEQ ID NO: 53 in the order from the 5' end to the 3' end. The complete sequence of the gene cluster was constructed on the pRK404 plasmid (it is published in the literature: Jiang Feng, et al. Cryo-EM Structure and Assembly of an Extracellular Contractile Injection System. Cell, 2019, 177(2):370-383e15, which is incorporated herein by reference) to obtain the recombinant plasmid pRK404-XkCIS (Fig. 1).

**Table 1. XkCIS structural gene sequences**

| | Base sequences | Protein sequences |
|---|---|---|
| XkCis1 | ATGTCTACAAGCACATCTCAGATTGCTGTTGAATACCCTATTCCT TCTTATCGTTTCGTTGTTTCCGTCGGAGATGAACATATTCCATTT AATAGTGTTTCAGGGCTGGATATTAATTATGAAACCATTGAATA CCGTGATGGCGTGGGTAGTTGGTTTAAAATGCCGGGTCAGCGTC AGCCGATTAATATTACCCTGCGTAAAGGTGTTTTTCCGGGCAAA ACTGAATTATTTGACTGGATTAATTCTATCCAACTTAATCAGGTT GAGAAAAAAGATATTTCCATTAGTTTAACCAATGACGCAGGCA CTGAATTATTAATGACCTGGAATGTTGCTAATGCTTTCCCTACCT CATTGACGTCTCCTTCCTTTGATGCCACCAGCAATGATATTGCC GTTCAGGAAATTACCCTGACAGCCGATCGCGTCACCATGCAGG CTGTTTGA (SEQ ID NO: 1; Locus_tag: AB204_RS00590) | MSTSTSQIAVEYPIPSYRFVVSVG DEHIPFNSVSGLDINYETIEYRDG VGSWFKMPGQRQPINITLRKGVF PGKTELFDWINSIQLNQVEKKDIS ISLTNDAGTELLMTWNVANAFPT SLTSPSFDATSNDIAVQEITLTADR VTMQAV* (SEQ ID NO: 17; NCBI Sequence ID: WP_047961455.1) |
| XkCis2 | ATGACAACCGTTACCAGTTATCCTGGCGTTTATATTGAAGAATT AAATAGCCTGGCATTATCCGTATCAAATAGTGCCACCGCCGTTC CTGTTTTTGCAGTGAACAAAGAAAACAAACAGATTAATGAAGA GACTGCACACCGAATTAATTCCTGGATGGATTATCTTAATCTGG TAGGCAACTTTGACAATGAGGATAAATTGTCAGTTTCTCTGCGG GCTTATTTTACCAATGGCGGGGGTTATTGTTATCTCATCAAAAC CCCAAATTTAGAAAAACTTATTCCAACCTTAGATGATGTCACTT TGCTGGTTGCGGCAGGTGAAGATATTAAAACAGCCGCGGCAAC TTTATGTCAGCCGGGAAAAGGGTTATTTGCCATTTTTGATGGCC CTGAAGAAGCGTTGACCATCGCGAATGCAGAAACAGTCAAGAA CAAATATGACGCCACCGCGTTCGCCGCAGTTTACTACCCGTGGC TGAAAACGGATTGGGCAAAAATCACCATTCCACCGAGTGCGGT TATGGCAGGCGTTTACGCCTCCGTCGATCTCTCCCGTGGCGTGT | MTTVTSYPGVYIEELNSLALSVSN SATAVPVFAVNKENKQINEETAH RINSWMDYLNLVGNFDNEDKLS VSLRAYFTNGGGYCYLIKTPNLE KLIPTLDDVTLLVAAGEDIKTAAA TLCQPGKGLFAIFDGPEEALTIAN AETVKNKYDATAFAAVYYPWLK TDWAKITIPPSAVMAGVYASVDL SRGVWKAPANIALKGGLEPAFLIT DEVQSAYNSGRAINMIRNFSNTG ATVWGARTLEDNDNWRYVPVR RLFNTVERDVKRAMSFAMFEPNS QPTWERVRAAISNYLHSLWQQG |
| | GGAAAGCTCCCGCCAATATCGCATTGAAAGGCGGACTGGAACC TGCATTTTTGATCACGGATGAAGTTCAGAGTGCATATAACTCCG GTCGTGCCATCAATATGATTCGGAATTTCAGCAATACCGGTGCC ACCGTTTGGGGAGCAAGGACGCTGGAAGATAATGACAACTGGC GCTATGTCCCTGTGCGTCGCTTATTCAATACCGTCGAGCGGGAT GTTAAGCGCGCTATGAGCTTTGCCATGTTTGAACCTAACAGCCA ACCAACGTGGGAACGTGTCAGGGCGGCAATCAGCAATTACCTT CATAGCCTGTGGCAACAAGGGGGATTGGCTGGCAGCAAAGAAG AAGATGCCTATTTCGTGCAGATTGGCAAAGGAATTACGATGAC GCCAGACCAAATTAATCAGGGACAAATGATAGTAAAAATCGGC CTGGCGGCCGTTCGACCGGCAGAATTTATCATCCTCCAGTTTAC GCAAGATGTAGAACAGCGTTAA (SEQ ID NO: 2; Locus_tag: AB204_RS00580) | GLAGSKEEDAYFVQIGKGITMTP DQINQGQMIVKIGLAAVRPAEFII LQFTQDVEQR* (SEQ ID NO: 18; NCBI Sequence ID: WP_047961454.1) |
| XkCis3 | ATGCCAGCTAATCTAAAAACGCCCGGCGTTTATATTGAAGAAGA TGCTTCCCTTGCATTATCGGTCAGCAACAGTCCCACTGCCGTGC CTGTTTTTATTGGTAAGTTCACCCCAAAAACTGCGGAGCCAACC CAAACCTGTATTCGTATCAGCAATTGGCTTGAATTTACTTCCTC TTTTTCTTTGGCACCAGTGACAGAAATTGTCATCCGCTCTAGTC TTGAAGCCTCTTCTGATGGCGCAACAAAAAAAGAACAGGCCAA AAAAGAAAAAGTCGAAAATAAAGAGACTGCAACCCAAGAAAC TCCCAAAGTTCAAACTCTCTCGGCAGCCGAAGAAGAACCCAA AAAATGGATTCACAATATTGAGAGAGTCAATCTATCTCAAGCCG TAGAAGCCTTGAAGCTCTATTTTCAAAATGGAGGCGGGGCTTGT TATATCTACCCATTAAACAATATTAATGGCGAGTCGGCTCTGGA CCCCATTCCTAAGGTGATAGAACAAAAAGGGGATATCACTCTG TTGGTTTGTCCAGAAGAAGATCAGGGTTATAAAAACAGAGTCT ACAGTGCAGTCAGCCCCCTATTGAATAGTGATAACAAAAAAGT GGGCTATTTCCTGATTGCAGATAGCCTTGATGGTAAATCGGTG TCTGGCATGGCAAATAGCACCAAGATCGCCGCCTATTATCCTC AGTTGGAAACAAACCTGAAATTCTCCACCTTGCCCGACGATCA GAACATTGTTATCAGTGGTTATGAGAACGCATCAGGCAAGGAC AAGCTGAAAACTCTGGATAAACTTAGGGAAACAGATAAAGCG CTGGCACAAGAAATTGATATCAGATTGCAAGAAATACAACAA GATAATCTCACCCTTTCACCCAGTGCTGCCATCGCAGGAATTT ATTGCCAAACTGATAGCCAGCGTGGCGTCTGGAAAGCTCCCGC CAACGTTGCACTCAGCGGAATCAAAGGTCTGACTGATAAAGTC GATGACGAACGGCAAGGCACCATGAATGAGAATGGCATCAAT GTCATCCGTTCATTTACCGGCCGTGGCTTGATGGTTTGGGGGG CGCGTACTTATGCTCAGAACGGTGAGATCAACTGGCGTTACAT TCCTGTCCGTCGCCTGTTCAATTCCGTTGAACGTGATATCCGTC AGGCACTGCGTGCCGTGTTATTTGAAACCAATAATCAACCGAC ATGGATGCGCGCCAAGGCTGCCGTTGACCAATATCTTTATGCC CTCTGGCAAAAAAATGCCCTGATGGGGAACAGCCCGGAAGAG | MPANLKTPGVYIEEDASLALSVS NSPTAVPVFIGKFTPKTAEPTQTCI RISNWLEFTSSFSLAPVTEIVIRSS LEASSDGATKKEQAKKEKVENK ETATQETPKVQTLSAAEEEPKKW IHNIERVNLSQAVEALKLYFQNG GGACYIYPLNNINGESALDPIPKV IEQKGDITLLVCPEEDQGYKNRV YSAVSPLLNSDNKKVGYFLIADS LDGKSVSGMANSTKIAAYYPQLE TNLKFSTLPDDQNIVISGYENASG KDKLKTLDKLRETDKALAQEIDI RLQEIQQDNLTLSPSAAIAGIYCQ TDSQRGVWKAPANVALSGIKGL TDKVDDERQGTMNENGINVIRSF TGRGLMVWGARTYAQNGEINW RYIPVRRLFNSVERDIRQALRAVL FETNNQPTWMRAKAAVDQYLY ALWQKNALMGNSPEEAYFVHIG QDITMSDADIKQGKMIMKVGLA AVRPAEFIVLQFTQDVAQ* (SEQ ID NO: 19; NCBI Sequence ID: WP_047961453.1) |
| | GCTTATTTTGTCCATATCGGTCAGGATATCACCATGTCTGATGC AGATATCAAACAAGGCAAAATGATCATGAAAGTAGGTCTGGC CGCAGTGCGACCGGCTGAATTTATCGTCCTGCAATTTACGCAG GATGTCGCTCAGTAA (SEQ ID NO: 3; Locus_tag: AB204_RS00580) | |
| XkCis4 | ATGGAAAGAATTCAACCGGGCGTGACCTTGACAGAAAGTATAA TTACCACAGGCCAGCAAGAGATACCCAGCGCAGTGCCGGTATT TATTGGCTATACCGTCCGCAATCAACAACAAGCAAAAACCGCA GTTCGTATCGACAATCTGGCGGAATATACCCAGCGATTTGGCGA CGATCATCTGATGGCATTGGCGGTAAGGCACTATTTCGATAATG GCGGACAACGGGCTTTTGTTTTGCCACTGTCAGGAAATATGCCC TCAGCAAACATGACCCAAGCGGAAGCCGAAGCACTGATGGCGG CATTAAGCTCTGACATGGTGCAAGAAGCCATTGGTGGGGAAAC GCAGATCACCCTGATTTTGGCACCGGATATCGCCCGACTGAATG ACAATGTGACTGATGAGCAACAAACTTTAGTCAATTTGTGGTC ACAGGGTTGGAGTACACTGCTGCAACTAAGCCAGATCCGCACG AATCTCTTTGTGCTGCTGGATGCGCCGGATGAGGTTGAACAGG CACGGCAATGTCTGGAAGGACTCTCGTCAGAGTATCGCCAATG GGGAGCCGCTTATTGGCCTCGTCTGGAAACCACTTATCAGGCTA CATCCAGTTCCAAAAATATTATGCTCTCCCCCACTGCCGCTATCG CAGCACTTATCCAACACACGGATAGCGAAGTTGGTGTATGGAA AGCGCCAGCCAATATTGCCCTGTCTCAGGTTATCCGGCCGGTTA AGTCGTATCTTCAAGGGAGTGTACTGTTTGACACCAATGGCACT TCGCTCAATGTGATCCGCACTTTCCCCGGCAAAGGCATACGCGT ATGGGGATGCCGCACACTGGAAAACACAGATAACACGCAGTGG CGTTACCTACAAACACGACGTCTGGTTTCTTATGTCACCGTACA TTTGGCGCAATTAGCCCGTATGTACATCTTTGAACCTAATAACG AACTGACGTGGATGAAATGCAAGGGACAAAGTTACAACTGGCT ACGGCAACTGTGGTTACAGGGTGGCTTGTACGGCTCACGGGAA GATGAGGCATTTACTGTGCTGTTAGGCGTCAATGAAACGATGAC CGAAGAGGATATTCGTGCCGGGAAAATGATAATGAAAGTCGCC TTAGCCGTATTGTCTCCCGCGGAATTTATCGAGATCAGTTTGGT ATTTGATACCCAAACGGGCGCGCTTTTGTCTTAA (SEQ ID NO: 4; Locus_tag: AB204_RS00575) | MERIQPGVTLTESIITTGQQEIPSA VPVFIGYTVRNQQQAKTAVRIDN LAEYTQRFGDDHLMALAVRHYF DNGGQRAFVLPLSGNMPSANMT QAEAEALMAALSSDMVQEAIGG ETQITLILAPDIARLNDNVTDEQQ TLVNLWSQGWSTLLQLSQIRTNL FVLLDAPDEVEQARQCLEGLSSE YRQWGAAYWPRLETTYQATSSS KNIMLSPTAAIAALIQHTDSEVGV WKAPANIALSQVIRPVKSYLQGS VLFDTNGTSLNVIRTFPGKGIRV WGCRTLENTDNTQWRYLQTRRL VSYVTVHLAQLARMYIFEPNNEL TWMKCKGQSYNWLRQLWLQGG LYGSREDEAFTVLLGVNETMTEE DIRAGKMIMKVALAVLSPAEFIEI SLVFDTQTGALLS* (SEQ ID NO: 20; NCBI Sequence ID: WP_047961452.1) |
| XkCis5 | ATGAACGATTATTACACCCCCGTGGTATCCCACCGTTTTATGG CGAGCTTTATTTTTAATGGCATTCCTGATCCACTGGATATCCGT TTTCAACGTATTTCCGGCCTGAACCGGGAACTTCAGGTCACCC AGCACAGCCAAGGCGGTGAAAACGCCCGCAATAACTATCTGG CTGAAAAAATCCAACACGGCACATTAACGCTGGAGCGCGGTG TGATGACGGTTTCTCCTTTGACCTGGATGTTTGACCGTGTATTG CGTGGAGAAAATATCGCCTATGCGGATGTGGTGATTATGCTGT TAAACGAAAATTCGCTGCCCTTGTCCAGTTGGACGCTGAGCAA TGCCTTACCGGTACGCTGGCAGACCAGCGATTTTGACGCGAAC AGTAATGCCATTCTGGTGAATACCCTTGAATTGCGTTATCAGG | MNDYYTPVVSHRFMASFIFNGIP DPLDIRFQRISGLNRELQVTQHSQ GGENARNNYLAEKIQHGTLTLER GVMTVSPLTWMFDRVLRGENIA YADVVIMLLNENSLPLSSWTLSN ALPVRWQTSDFDANSNAILVNTL ELRYQDMCWLGVKV* (SEQ ID NO: 21; NCBI Sequence ID: WP_047961451.1) |
| | ATATGTGCTGGCTGGGAGTCAAAGTATGA (SEQ ID NO: 5; Locus_tag: AB204_RS00570) | |
| XkCis6 | ATGACCGTCGAAATCAGAGAATTAATTATTCAGGCAAAAGTG GTTTCATCATCACCAATACTCCCAACACAAGCAACTGAATCAC ATCAACAAAGCCATTCTTTGATTCAGGAAAGTCTGGATGAGGC AGCTTTAGTTGAAAAAATTAAACGCGAAGTGCTGGCTGCATTA CGCGATGAGGAAGGATGGCGCCCATGA (SEQ ID NO: 6; Locus_tag: AB204_RS00565) | MTVEIRELIIQAKVVSSSPILPTQA TESHQQSHSLIQESLDEAALVEKI KREVLAALRDEEGWRP* (SEQ ID NO: 22; NCBI Sequence ID: WP_047961450.1) |
| XkCis7 | ATGAGTTTAATTGAACGTGGTTTAGCGAAGCTGACGATTAACG CTTATAAAGACAGGGAAGGAAAAATACGGGCTGGCACCATTC AGGCCATGTATAACCCTGATTCCCTGCAACTGGATTACCAAAC GGATTACCAACAATCCCAGGCAATTAATAGCGAAAAGCAGAG CAGCATTTATGTACAGGCCAAGCCTGCCGGGTTATCCCTTGAG CTGATTTTTGACGCCACAATGCCGGGCAACAAAACCCCTGTTG AAGCTCAGTTAATGCAGCTCAAGCAGTTGTGCAGCGTGGATGC CACCAGTAACGAAACGCGGTTCCTGCAAATCAAATGGGGAAA AATGCGTTGGGAAAACCGCGGCTATTTTGCTGGCAGGGCGAA AGGTTTGTCTGTGAATTACACCTTGTTTGATCGTGATGCCACGC CATTGCGGGCGCGGGTCATCCTGACATTGGTGGCGGATGAAAG TCTGGTCTTACAGGAGACAGAGCAAAACCTGAAATCGCCGGC GAAAATTGCCTTACGGGTACAGGATGGTGTTTCGCTGGCCCTG ATGGCGGCAAGCACCGCATCAACGCTGTCTGGTGGTGTTGATT ACCTGACGCTGGCCTGGCAAAACGGTCTGGATAATCTTAACGG ATTTGTACCGGGTGAGATATTGCAGGCCACCAGAGGAGATGCC TGA (SEQ ID NO: 7; Locus_tag: AB204_RS00560) | MSLIERGLAKLTINAYKDREGKI RAGTIQAMYNPDSLQLDYQTDY QQSQAINSEKQSSIYVQAKPAGL SLELIFDATMPGNKTPVEAQLMQ LKQLCSVDATSNETRFLQIKWGK MRWENRGYFAGRAKGLSVNYT LFDRDATPLRARVILTLVADESL VLQETEQNLKSPAKIALRVQDGV SLALMAASTASTLSGGVDYLTLA WQNGLDNLNGFVPGEILQATRG DA* (SEQ ID NO: 23; NCBI Sequence ID: WP_047961449.1) |
| XkCis8 | ATGAGCCATCAACTGAAAATCATTGCCGATGGCAAGGCACTGT CTTTGCTGGCCGCCGTTGATGTCGATACTTACTGCCGCGTTAAC AGCATCCCTTCCGCAACGCTGAAACTCAGCCTGCCGGACAGCC CTCTTTCCTCTTTCAGCCAAACGGATGTACAGGCAGAACTGGC CCATTGTCAGGCAGGGAAAACCCTGCGTCTGGAATTGACTGAC AGCGGCAGAAAATCAGTGCTGTTTAACGGCCTGATTACCCGTA ACTCTTTGAAGATAAAAAACAAGCAATTATTGCTTACTTTGGT TGTCCGACATCGGTTGCAACTGATGGCAGATACTCAGCACTCA CAACTGTTTAAGGCCAGTAATGAAAAAACCATTCTGAACACCT TATTGAATCAGGCGGGTATCAAGGCTCGTTTTGAGCGGATAGA CGCACTGGATCAAAAACATGAACAGATGGTGCAATTTCGCTGT TCAGACTGGCGTTTTCTGTTGTGCCGACTATCGGCAACCGGCG TATGGCTGTTACCCACGACAGAAGGTGCTCAGCTTGTCCAGCC TAACACCCTGAAATCCAACCCAACCCATACCCTGAAAAGCCGG GGAGATGAGAAAAAAGATATCATCGTCAAGGATGCCAACTGG CAGTTTGATAACCATCTTAACCCTGCTTCGCTGGAAGTCAGCG GCTGGGATATCAGTAAACAGCAAGAATTATCAGGTGGTCGCC ACGGCAGTGTTGCCATGGGCAAAGCCGCACTTTCTCCTGACAG | MSHQLKIIADGKALSLLAAVDVD TYCRVNSIPSATLKLSLPDSPLSSF SQTDVQAELAHCQAGKTLRLEL TDSGRKSVLFNGLITRNSLKIKNK QLLLTLVVRHRLQLMADTQHSQ LFKASNEKTILNTLLNQAGIKARF ERIDALDQKHEQMVQFRCSDWR FLLCRLSATGVWLLPTTEGAQLV QPNTLKSNPTHTLKSRGDEKKDII VKDANWQFDNHLNPASLEVSG WDISKQQELSGGRHGSVAMGKA ALSPDRLASLNKTSWDIRYSSPLT TQESGYLAQGLLLNQRISGVTGE FLLKGDGRYQLGDNIQLTGFGSQ LDGTASITAVRHRFNRRIDWETT LSIGLPQEYLPMLPDATGLHIATV AKYQQDKAALNRIPIILPVLNRPN EFLWARLGKPYASHESGFCFYPE |
| | GCTGGCGTCTTTAAATAAAACAAGCTGGGACATTCGCTACAGC AGCCCGTTAACCACCCAAGAGAGTGGCTATCTGGCACAGGGA CTGTTGTTGAATCAGCGCATTTCTGGTGTTACAGGCGAATTCCT GCTTAAAGGTGATGGACGTTATCAGTTAGGGGACAACATTCAA TTAACGGGATTTGGTTCACAGCTGGATGGAACAGCAAGCATTA CTGCGGTTCGCCATCGTTTTAACCGGCGAATTGATTGGGAAAC CACTTTAAGCATTGGCTTACCACAGGAATATCTGCCGATGCTA CCCGATGCCACCGGACTCCATATTGCGACAGTCGCGAAGTATC AGCAGGACAAGGCAGCCCTGAACCGCATTCCGATTATTCTGCC GGTACTGAATCGTCCGAATGAATTCTTGTGGGCCAGATTAGGG AAACCTTACGCCAGCCACGAAAGTGGTTTCTGTTTTTACCCAG AACCCGGTGACGAAGTGATTGTCGGCTTTTTTGAAAATGATCC TCGTTATCCGGTGATTCTGGGGGCAATGCACAACCCGAAAAAT AAAGCGCCTTTAGAACCCACCCAGGATAATCAGGAAAAAGTC TTGGTGGTGAAAAAAGGAGAAATCCAACAACAAATCATGATT AATCGCAAAGAGAAAACTATTCAAATGAGTGCAGGAGAAAAC CAGATAATGCTCCAGCAGGATAAAGATATTTCGCTGTCAACGA AAAAAGAATTAACACTGAAAGCGCAGACCATGAATGCCGAAA TGGAGAAATCACTCAATATTTCTGGCAAAAACAGTGTTGAGAT CAAAGGCAGCAAAATTAATCTTACCCAATGA (SEQ ID NO: 8; Locus_tag:AB204_RS00555) | PGDEVIVGFFENDPRYPVILGAM HNPKNKAPLEPTQDNQEKVLVV KKGEIQQQIMINRKEKTIQMSAG ENQIMLQQDKDISLSTKKELTLK AQTMNAEMEKSLNISGKNSVEIK GSKINLTQ* (SEQ ID NO: 24; NCBI Sequence ID: WP_047961448.1) |
| XkCis9 | ATGGAAAACCAAATACTGACACAGCTTTATGGTCGTGGCTGGG CTTTTCCTCCCGTCTTTTCCCTTGAAAAGGGGGTGGAAATGGCT GAGGGGACAGAAGATGTGCGCCAAAGTTTGCATATTCTGTTCA GTACTGAGCCGGGGGAACGCCTGATGCGCGAAAATTACGGCT GTGGATTAAATGATTTTATGTTTGAAAATATCCGCAACGAACT GTTTGCTGAAATTGAATCCCATATCCATGACAGCGTATTACGT TATGAATCACGGGCAGATATTACCGATATCCAAGTTCGTCAGT CGCCAAATAGCAAAAATACCTTGCAAGTGCAGGTGATGTATCG CCTGCGGGGCAGCGATATCAATCAGCAAATTCAAGGAACACT GGCGCTGGGTGAAGGCCAGGGAATGGAGGTGGTATGA (SEQ ID NO: 9; Locus_tag:AB204_RS00550) | MENQILTQLYGRGWAFPPVFSLE KGVEMAEGTEDVRQSLHILFSTE PGERLMRENYGCGLNDFMFENI RNELFAEIESHIHDSVLRYESRAD ITDIQVRQSPNSKNTLQVQVMYR LRGSDINQQIQGTLALGEGQGME VV* (SEQ ID NO: 25; NCBI Sequence ID: WP_047961447.1) |
| XkCis10 | ATGAGTGAACCCATTGTAACTGACGGCGACACATTACAGTTTG AGCCCCAATTTGAGAGTCGAAAGGTGAACATTCCCGGATTGAT TAAAATCAATGGAACAGGTCATGCACAGGTCAACGGCAAAAA AGTTTGCATTTTGGGAGACGAGAAACGGGTTACTGTTTCTGCA ACTTATACCACAACAACTCATATGACACCGGGAACTGGCACCA TTACTATCACGGCGTTAGATAGCAGCCAGCAAGCCCTTCAATG TACCAGTGATGCTGCTTTAATTATCAAAGGGAAAAAATTTACG GCCATATTTACGCCTCAATCACCAGCCATAAATGGTAATGTGA ATCCACCTCAACCTGATCCTAATTACCCAAGCCCTTCATCAGG CAAAGGCAGTTTTATCACCCAACAAAACTTTGCCACTGTGAAT TAA (SEQ ID NO: 10; Locus_tag: AB204_RS00545) | MSEPIVTDGDTLQFEPQFESRKV NIPGLIKINGTGHAQVNGKKVCIL GDEKRVTVSATYTTTTHMTPGT GTITITALDSSQQALQCTSDAALII KGKKFTAIFTPQSPAINGNVNPPQ PDPNYPSPSSGKGSFITQQNFATV N* (SEQ ID NO: 26; NCBI Sequence ID: WP_047961446.1) |
| XkCis11 | ATGGAATTAACTGAGTTAAATAAAAAATTGTCAGATTTGGTGCC GATGAGCGATTTTAAATTAGATAGCCGAACCAGTTTGCAATTAC TCCAATATATTGAGGAGTACACTAAAATAATTCCCTTTAATCAA ACCGATAAGCATTGGAATGATTTTTTCTTTAAGAAAGATAGTAC GCCAGAGAAACTGGCCGAATTATATCAAGGGAAAATCGAAGCC AATGGGAATTTATTACCTCATCAAGCTTTTCTACTAGCGGTATTA CGATTACTGGAAACACCAATATCCTTATTGAATACCTTACCGGCG GCGCATCGCAATCTCTATTATCGGGCGCTTTTGGGGCTATCTCC CCGTCCGGCTCGACCTGATCAAGTTGCCTTATCTGTAGAACTGA ATACCACAGTCACAGAACAACTACTCACTAAAGGCACTTTATTT GAAGCAGGGCAAGATGAACACGGCAATGCCTTGCAATATGCGC TGGATACCAGTTTGCTGGCAAACCGTGGCTATATTAGTGAATTG CGCTGGCTCCGTAAAAAAGAACAACAGCAATGGATCACCGCGA CTCCCTGGAATTCACAGGCACAATTATCATTGCCATCTGGTGGA ATACGGTTATTCAGTGAAACTGATTTGGATAAATCGGTACTGGG CGGGATGTTGATCACTTCCAAACATCTGGCGATGGAAGAAGGA GCAAGAGAGATAACGATTACCTTTGATAAGGACATGGATAGCC AGAATTTGGTGGCACAAATCAGAAGTGGTAATCAATGGCTAAC TTTACAAGTTGAGAAAAATACAGCTGAGAGAAATAAAAAAGAA GTCATAATGATGCTGGCAGATAAAGAGCCAGCAACCCGTGCAC CAGAGGATCTGGATAACCTGATTTTCAGTGAGCCAGTACTCAG GCTACAGGGAAAAAACAGCCAACCACTGCCTACAGTCACCGC ACTAAAGGTGCAAAAAACAACTATAGATAAAGCTTCATTCAAG ACATTTCACTTAACCCCCTTTGGTTATAGCCCTGAAATTGAACC ATTGGAAGAAAACCCCGCATTATACCTTGGTATCACTGACGTCA AGCCGGGACAAACCCTATCGCTGTATTGGAAATTAAAATCGCCA CAGCAACCCGAAAACGTTTTCTGGTATTACCTGAATAATAAGAA TCAATGGGTTGCATTAGATGCGTGGGTCAAAGATGAAACCAAAT ATCTGTATCAAGATGGCATCTGGCAAGTTGTATTGCCTGCGGAT GCTTCTGCTTCTGCTGAAGAAATTACCCGGATGCCAACAGGGC GCTATTGGTTAAAGGCAGTCATGGTTATCCCCACACACAAAGG TTCATGGGGAAAAACTCCTTGGTTAGACGGCTTGATCTATAACG CCATGACAGCGACATTAACGAATGCCGATAGCATCAGCAACAG CCATTTCTTGACGCCATTACCGGCAGACACTATTCAACGTCCCG TTGAGCCTATCATGGAGGTGGCTTCGATCAGTCAGCCTTGGCCG TCGTGGAATGGGCGTGTACCGGAATCCTACAGCGCTTTTTTTGC ACGGGGAGCTCAACATCTGTCCCACCGTAACCGCGCCTTAACC TGGGGCAATATGGCGACACTGTTAAAAGAGCGTTATGTCAGCAT TTTTGATGTTAAGTACCCCGGCCATGATGAACTCACGCGAGTCC CCGCACTGGAGAAACAGCAACTGACCATCATTCCGGCTAACCA ATACAACGACAGCGATGATCCTTTGCGTCCGGTGTTAAACCCTG CCCGCCTGCAAGGAATGGCCGAATGGTTACAGCAAAAAGGCTC TCCATGGGCTGAGATTGAGATCAGCAATCCTCAATACATTGATG | MELTELNKKLSDLVPMSDFKLDS RTSLQLLQYIEEYTKIIPFNQTDK HWNDFFFKKDSTPEKLAELYQG KIEANGNLLPHQAFLLAVLRLLE TPISLLNTLPAAHRNLYYRALLGL SPRPARPDQVALSVELNTTVTEQ LLTKGTLFEAGQDEHGNALQYA LDTSLLANRGYISELRWLRKKEQ QQWITATPWNSQAQLSLPSGGIR LFSETDLDKSVLGGMLITSKHLA MEEGAREITITFDKDMDSQNLVA QIRSGNQWLTLQVEKNTAERNKK EVIMMLADKEPATRAPEDLDNLI FSEPVLRLQGKNSQPLPTVTALK VQKTTIDKASFKTFHLTPFGYSPE IEPLEENPALYLGITDVKPGQTLSL YWKLKSPQQPENVFWYYLNNKN QWVALDAWVKDETKYLYQDGI WQVVLPADASASAEEITRMPTGR YWLKAVMVIPTHKGSWGKTPW LDGLIYNAMTATLTNADSISNSHF LTPLPADTIQRPVEPIMEVASISQP WPSWNGRVPESYSAFFARGAQH LSHRNRALTWGNMATLLKERYV SIFDVKYPGHDELTRVPALEKQQ LTIIPANQYNDSDDPLRPVLNPAR LQGMAEWLQQKGSPWAEIEISNP QYIDVKINYNVIFKPDVNEDFGY RQLRQQLSETYMPWSVDEKRPV TLNNSISYYQLLATIQQQPLVERV TCLTLRRSEAAKDSDITASVEAKD NEVLILVWEADDKLQCRGNNYE * (SEQ ID NO: 27; NCBI Sequence ID: WP_047961445.1) |
| | TAAAAATCAATTACAACGTGATTTTTAAGCCGGATGTTAACGAA GATTTTGGTTATCGCCAGCTACGACAGCAACTCAGCGAGACATA TATGCCCTGGAGCGTCGATGAGAAACGCCCCGTCACGCTGAAT AACAGCATTAGTTATTACCAATTACTGGCAACTATCCAACAGCA GCCGCTGGTTGAGCGGGTCACTTGTCTGACGCTGCGTCGGTCGG AAGCGGCTAAAGACAGTGATATCACCGCATCAGTGGAAGCCAA AGATAATGAAGTGCTGATTTTGGTCTGGGAAGCAGATGACAAG CTGCAATGCCGAGGAAATAACTATGAGTAA (SEQ ID NO: 11; Locus_tag: AB204_RS00540) | |
| XkCis12 | ATGAGTAATCAAGATGCACTATTTCACAGTGTGAAAGACGATAT TCACTTTGATACCTTGCTGGAACAGGCTCATCAGGTGGTTGAGC AACAGGCAGGAAAATTGTGGAGCGACACAGCGGAACACGATCC CGGTATCACCTTTCTGGAAGGACTCAGTTACGGTGTGTCAGATT TAGCTTACCGTCATACTCTTCCTCCGACAGACTTACTGACCCCTG CCCCTAATGAGCAGAAACAGCAAGATGGAATTTTTCCCGCTGAA TTTGGCCCCCACAATACACTGACTTGTGGGCCAATCACCACTGA TGATTATCGGAAGGCATTACTTGATCTGCATAGCAGTGACTGGA CAGGAGCAAAGCCAGAGTCAGAACAGGACAAAGGGGATTTTCT GTTCCGTAACGTGCAACTGGTACGTGAACCGGAAACACAACGCT ATACCTATTGGTATGATGCCACCAAAAGGGAATATAGCTTTGTC GAGCGTAAAGATGCGAAAAAGTTTACCCTGCGGGGAAATTATT GGCTTTATCTGGAACCGACCCGTCGTACTCAGGAGAATTTGGAT AATGCTACCCAACAATTAATCGATTTTTTGACTCAAAACCGCAA TATTGGTGAATTGGTCAGTCAGAGAATCTGGACAAAACCGGTTG ATTTTCCGCTATTACTGGATATTGAACTGGATGACGATGTGCAA AATGTCGCTGGTATTTTTGCTGCCGTCTATAGCACAGCAGAACA GTATCTGATGCCTGAGGCGCAGCGTTTCAGTACCGAAACACTAC AAGATGCGGAAATGCGTAATGATGAAATCTTTGAAGGACCACG ATTAGAACATGGCTGGATCCCTGAATTGCCGACAGCCCGTGATT ATACCAAACGGCTCAAGCTCAATCTTAGCCGGCTGGTCAATAAG CTGCTTGAGATTAAGGGAATACAGAATGTAAATCGTCTTCGACT GAATGAAAATTTTGATAAGACCCTGATTGAGCCGATTAAAGG GGATAGCTGGTCATGGTCAATAAAAGAAGGCTACTACCCTCGTT TGTGGGGTAAAGATCCGATTCATCAATTGGCACAACATGATGGC CCACTGAGGGTGATCGCCAAAGGTGGGATCAGCGTTACGGTTGA TGAAAACAGCATTCGAAAAAGTTTATCCTTATCCCATCCTCCTCT TATTCAGAATAAACCTGTGCTATTGGCTTATGGTCGGCATCGCG ATGTGGGTCGCTATTACCCCGTCAGCGATACGTTGCCCGCTTGCT ACAAATTACAGCAGCCTTTATCTGAAAGTGAACATGCGCAACGC CTGTTATCACTTCATCAATTTATGCTGCCATTTGAACAATTGTTG GCTTGCGGCTGCCAGCAAATTGCCATGCTGCCAAAATTGTTGGC TTTTAAGCGTGAGGGATATGAAGTTTGGGGTGATCAATGGCCAT TTAAGCCGGGTTCTGTCAATGATGATGCCCACCAAAATTACGCT | MSNQDALFHSVKDDIHFDTLLEQ AHQVVEQQAGKLWSDTAEHDPG ITFLEGLSYGVSDLAYRHTLPPTD LLTPAPNEQKQQDGIFPAEFGPHN TLTCGPITTDDYRKALLDLHSSDW TGAKPESEQDKGDFLFRNVQLVR EPETQRYTYWYDATKREYSFVER KDAKKFTLRGNYWLYLEPTRRTQ ENLDNATQQLIDFLTQNRNIGELV SQRIWTKPVDFPLLLDIELDDDVQ NVAGIFAAVYSTAEQYLMPEAQR FSTETLQDAEMRNDEIFEGPRLEH GWIPELPTARDYTKRLKLNLSRLV NKLLEIKGIQNVNRLRLNENFDKT LIEPIKGDSWSWSIKEGYYPRLWG KDPIHQLAQHDGPLRVIAKGGISV TVDENSIRKSLSLSHPPLIQNKPVL LAYGRHRDVGRYYPVSDTLPACY KLQQPLSESEHAQRLLSLHQFMLP FEQLLACGCQQIAMLPKLLAFKRE GYEVWGDQWPFKPGSVNDDAHQ NYAYELKKVLKEIAHDNDHELDI VNYLLGYFGTQRAPRTFTTNISDF RAVQQGYLAQQPTLTYHRANIRI DQVSSLQKRIAARMGLGGELFKP KPDLSKLPFYLVEHRALLPVKPNS QFDEEQKPDSVTEERNYQNDQYY LVIKQAGIDGKLIQGQVIDLVLYE GEQNENRFTVRGQMITKTTGDQF WLDVSNSTQLEYNLERVMTAAK GKKLVWQNSPVWMEDMNYRLA YDSDQSSLPENQRRLTRTAQTPFP ALIAAGNEITLTKQLGGVGLEQES SNESEKLYAKVVSFDRIKGTLIIER |
| | TACGAATTAAAGAAAGTACTAAAAGAGATTGCACACGATAACG ATCACGAATTGGATATTGTCAATTATCTGCTCGGATACTTTGGCA CTCAGCGAGCACCACGTACCTTTACCACCAACATCAGTGATTTT CGTGCCGTGCAACAAGGCTATCTGGCTCAACAACCGACATTAAC TTATCACCGAGCCAATATTCGGATCGATCAAGTTTCGTCGCTAC AAAAGCGCATTGCTGCCCGTATGGGGTTAGGGGGTGAATTATTT AAACCAAAGCCTGACTTGAGCAAACTGCCTTTTTATCTGGTAGA ACATCGGGCATTGCTGCCAGTCAAACCCAACAGCCAGTTTGATG AAGAGCAGAAGCCAGATTCGGTGACAGAAGAAAGGAACTATCA AAATGATCAATATTACTTGGTGATTAAGCAAGCCGGTATTGATG GAAAACTGATACAGGGACAGGTAATTGATTTAGTTCTCTATGAA GGAGAACAGAACGAAAACCGATTTACCGTGCGTGGTCAAATGA TCACTAAAACCACAGGAGATCAGTTTTGGCTGGACGTGAGCAAC AGTACTCAACTGGAATACAATCTGGAACGGGTAATGACCGCCGC CAAAGGGAAAAAACTCGTCTGGCAAAATAGCCCGGTATGGATG GAAGATATGAACTACCGGCTGGCCTATGATAGTGATCAATCCTC ATTGCCTGAAAACCAGCGGCGCCTGACCCGCACAGCACAAACC CCCTTCCCGGCCCTGATTGCCGCAGGTAATGAAATTACCCTGAC AAAACAGTTGGGGGGAGTGGGTTTGGAGCAAGAATCTTCTAAT GAATCAGAAAAATTATATGCAAAAGTTGTCAGCTTTGATCGCAT CAAGGGAACTTTGATTATTGAGCGTCAGAAACATTCTACACTGG CATTCCCTGCCGCAGAAGAAGCCTGGCGGCACAGTTGGCATTTT TCGGGTAAGGAATATGAAAATACGGACCGTTTCTCATTTGTGAT TAGCGTGGTGGTAAATAGTGATTTAATTAAATCACGTGAGCTCA AAGTTGATCCATATCAATTGGAAGAGTGGGTTAAGGAAACTATT CTCACCGAATTCCCTGCCCATATTTCCATGATTATTCACTGGATG GATCGGGAGCCATTTTTAAACTTCGGCAATACGTATCAACGTTG GCAAAATAATGGCGCACCATTAGGGGATGCCGCTTATTCGATTC TGGAAAGTTTGACGCTGGGTAAATTACCTTCCGCTTTCAAAGGA ATTGGGTCAATGCGTATTGCCACACCACATCAGAGAGAGGAAGT TGTTGGTAAGAATGGTGAACAATGGAATACCAATAGAATAAAA CAAAACGAATTATTCTATATTCCGAAAGAGAAAGAATAA (SEQ ID NO: 12; Locus_tag:AB204_RS00535) | QKHSTLAFPAAEEAWRHSWHFSG KEYENTDRFSFVISVVVNSDLIKS RELKVDPYQLEEWVKETILTEFPA HISMIIHWMDREPFLNFGNTYQR WQNNGAPLGDAAYSILESLTLGK LPSAFKGIGSMRIATPHQREEVVG KNGEQWNTNRIKQNELFYIPKEK E* (SEQ ID NO: 28; NCBI Sequence ID: WP_047961444.1) |
| XkCis13 | ATGAAAGAAATTCGTTATTCTGCAAAAATACAAGAACAACAAA CATTATCTGACGCAAAAGGTGTTGGACCTGATATTGATAAATTA AAAGATAAGTTTAAAGAAGGCAGTATTCCCTTACAAACCGATTT CAATCAATTAATTGATATTGCCGATATTGGGCGTAAAGCGACTG GACAGGCTCCCAATCAAAATGGCCCGGCAAAAGGAATGCGTTT GGGCGAAGATGGGCTTTTACAGTTAAAGTTAGATGAGAAATAC GCTCATGCAAATTACAGTCCATTAAGATTGCATGATGATGCATT GGTTGTAGGAGTAGGCAATGGTTTGATAAATTTTAAAAAATCAA ATGAAATAGGTGTTAATGATGGAAATGGTATTAGGATAGATGAT AATAAGGTTGCAGTAGAACTGGCTGATAATAAAGGTTTGGTTGT | MKEIRYSAKIQEQQTLSDAKGVG PDIDKLKDKFKEGSIPLQTDFNQLI DIADIGRKATGQAPNQNGPAKGM RLGEDGLLQLKLDEKYAHANYSP LRLHDDALVVGVGNGLINFKKSN EIGVNDGNGIRIDDNKVAVELAD NKGLVVDSNGIAIKPGNGVKFSSD GALTVNSADSTIKVDGSGIKVATG WGVKVGGEGLDVNVRTDGGIGN DSSGIYVKTGNGIKLDNDQVAVE |
| | TGATAGCAATGGTATCGCCATAAAACCAGGCAATGGGGTGAAG TTTAGTTCAGATGGTGCATTAACAGTAAATTCGGCTGATTCAAC TATTAAAGTTGATGGCTCAGGAATAAAAGTTGCTACAGGTTGGG GAGTTAAAGTTGGTGGTGAAGGTTTGGATGTAAATGTCCGCACG GATGGTGGTATTGGTAATGATTCGTCAGGTATTTATGTTAAAAC AGGTAATGGTATTAAGTTAGATAATGATCAAGTTGCAGTAGAAC TAGCTGATAATAAAGGTTTAGTTGTTGATAGTAATGGTATCGCC ATAAAACCCGGCGATGGAATGCAATTTAGTTCAGATGGTGCATT ACAGTTAAAATTAAATAAGAATTATGCCGATAGATTTTATAGCC CTTTAATATTGCGTAATGATGTATTGGCTGTAGGTATAAGCCAT GGTTTGACAACTTATCAAGGATCATATGCAATAGGTGTTAATCC AGGAAATGGTCTTAGGATAAATGACGATAGTGTTGCAGTGAAA CTAGCTGATAATAAAGGCTTGGTTGTTGATAGCAATGGTATCTC CATAAAACCAGGCAATGGAATTAAGGTAGATGCTGATGGTGTG GCTATCGATCCTAATACTGTATTGCCACGTGGTATGATTATGATG TTTTCTGGTAATAGCGTACCAGAAGGTTGGGCTATTTGTGATGG CTATAATAATACACCAGATTTTCTAAATAGATCTATTGAGGGAA ATACTAGGAATATTAAAAGTAAAAATGGTATAGATATGACTATA CAATATACTGCAATATATATCATGAAATTATAA (SEQ ID NO: 13; Locus_tag: AB204_RS20710) | LADNKGLVVDSNGIAIKPGDGMQ FSSDGALQLKLNKNYADRFYSPLI LRNDVLAVGISHGLTTYQGSYAIG VNPGNGLRINDDSVAVKLADNKG LVVDSNGISIKPGNGIKVDADGVA IDPNTVLPRGMIMMFSGNSVPEG WAICDGYNNTPDFLNRSIEGNTRN IKSKNGIDMTIQYTAIYIMKL* (SEQ ID NO: 29; NCBI Sequence ID: WP_053067800.1) |
| XkCis14 | ATGATGACCAACACTGATAAATATGGTGTTAATCTTATTAATAG AATAACTATCTCCATTGATATGTTATATTTTAACGATGATAACAT AATGGAGAACTGCTCATCTATATTTAATAGAAAACTAAAAAATA TCATCAATAAAAATATCAAAAACCTGACATTGAATGTAAATGGG AAGTTATCTCTTGAAAGGGTAACTCTGGATCTGGGAAATATTGA AATTGATTTATTTGAAAGGGAAATAACAAGAAGATTGGAAATA GAATTAAAAAAGAACTTAAGAAATCATTATGGTTACTCTCAATG TATTTTAAATAAAGAGAAAATATATCCACACCAATCAGAAAAA GAAAATAGATTTTTATTAATAAAAGAAATAGATCATTACTTACA TAAGGGAAACTGGTTATCTGATCACAGAAAGCATCATATTACAA ATACTGTGCATATTAATAATGAATTATGGCAACAGATTAACCAC CAGCCACAACAATGGCTACCTATGTTAGCCAAACACTGCTTACA GCCTGCGGGGCTAAGTCGCTTAATTCAACTCTGCCAGCCAGACG TTCTTGCTATGCTTTGCCAGTTATTTACTGAAACAACAACATCCG AATTTGTTAATTCGCAAGCGCAGGTAATTACAGATAAAAAAGTG AAACCAGAAAAATTGAGTTTGGCTGCGGAATATTATCTGCAACA TCAGGTGAAATATCCCCATGCCACACAACAAGCACTTGCACAAC AAACTCAAAATAAGCAAACACCGACAATAAATATTATTCAGAA TAATAACAGTCTATTAAAACAGACTTACAATGAAACTGATATGC AGATGGATACAGTACTGCCATTTGCAACATACCATCAGCCAACA GCTACGCAACAGCATTATGGTCTTATAAAAGAGGCGGTAAAAC AAAGTCTTCCAACTTTACAAAACAGAGAGCAACATAAGGAATT ACCGAGATCAACTTCACCAGCGAAAATATCATCCATACAACCCA | MMTNTDKYGVNLINRITISIDMLY FNDDNIMENCSSIFNRKLKNIINKN IKNLTLNVNGKLSLERVTLDLGNI EIDLFEREITRRLEIELKKNLRNHY GYSQCILNKEKIYPHQSEKENRFL LIKEIDHYLHKGNWLSDHRKHHIT NTVHINNELWQQINHQPQQWLP MLAKHCLQPAGLSRLIQLCQPDV LAMLCQLFTETTTSEFVNSQAQVI TDKKVKPEKLSLAAEYYLQHQVK YPHATQQALAQQTQNKQTPTINII QNNNSLLKQTYNETDMQMDTVL PFATYHQPTATQQHYGLIKEAVK QSLPTLQNREQHKELPRSTSPAKIS SIQPSNGIQTPSSQSVLTISNAGCMI LWPLLPIFFRTFDLLEKNQFISLEA QREAVCLLDWLIWEEEEIPAWRL TLNKILCGLPIHDNARWRTPKPEQ QIAINQWLEKIIEQLPAWKKMGIN DVRHLFLQRSGELSELNGIKNIHIK PEVYDALISEWAWPMNIASFSWL QHPITITWS* (SEQ ID NO: 30; |
| | GTAATGGAATTCAGACTCCATCATCACAATCCGTTTTAACCATC AGCAATGCCGGTTGCATGATTTTATGGCCGTTATTGCCCATATTT TTCCGTACCTTTGATTTACTGGAAAAGAATCAATTTATCAGCCTT GAAGCACAAAGAGAGGCGGTCTGTTTACTTGATTGGCTGATTTG GGAAGAAGAGGAAATACCTGCATGGCGATTGACTCTCAATAAA ATACTTTGTGGATTACCCATACATGACAACGCGCGATGGCGTAC CCCAAAGCCAGAACAACAAATTGCCATCAACCAATGGCTGGAA AAAATCATCGAACAACTTCCAGCCTGGAAAAAAATGGGAATAA ACGATGTGCGACACTTATTTCTGCAACGTTCCGGGGAATTAAGT GAGTTAAATGGAATAAAAAACATCCATATTAAACCCGAAGTTTA TGATGCACTGATAAGCGAATGGGCGTGGCCAATGAATATCGCG AGCTTTAGCTGGTTGCAACACCCTATCACCATAACGTGGTCATA A (SEQ ID NO: 14; Locus_tag:AB204_RS00525) | NCBI Sequence ID: WP_047961443.1) |
| XkCis15 | ATGAATACAGCGCCTGTTTTCCCTGATTTATTTCAGCAACATAAT GATCCTCATTTAATGTTCTTATATAGCCAACTGGAACGCATCGAT CTCGTTCTGCAATACCATTTCCATTGCATATCAGGCCAGAAAGG TGACCTCCTTGATGAGTTCTTGTTAAGCGAAGAAGATGTGATAA ACAGACTGGATAACCCTCTGGGAAAACCTCATTGGCTCACCGAT GATTGCCTGACAATACCGCACACCGTCACGCCCGTGGCATCCCG ATTAACTGACCTGATTACACATTTTGAACTGACTGATTTTGAAA GGGATGTTTTGTTGTTAGGTTTACTGCCCCATTTCGATAACCGCT ACTATCACCTGTTTGCTCTGGTTCAGGGAGAACAACAATCTCGG CTGCCCGCTTTTGCGTTGGCTTTGGAACTGTTTTGCCACTCAATG CTGGAAAAACAGGCACAGCAGGCCAGTTTTTTACACCAGGCTCC CTTGGTTGGCTGCCAACTGTTATCTATTGATAGTCGTCAGAAAA CGTTATCCTGGCTCCAGACACCCTTTATCACAGACAGCAGCGTA TATCATTTTTTACTTGGGCATCACTATATTGCACCTGCTCTGGAA CATTGTGTTGAGTGGATAACGCCCACAGAAATCGGCAATTATCC AGCAGGATTAAAACAAGCGTTGGGTAATGTACTGTTATCTGATC ATGATGATATCCGGCCCGTTATCTTACTGCGGGGCATGGCCGAT AGTGCCAGATCCCATGTCGTTGCTAATATGATGGCATCAGAACA ACGGCAAACGCTGCTTGTGGATATCGCCAGCTTAGCTGATAACG ATGACAACTCCCTGTTTTGCCATATAAAAGCCATTTTGCGAGAA ATTCGTATGCGTGGTGCCTGTTTATTGTTACGAAACTTTTGCTTA TTTGCAGAACAGAATAAACACTTGCTGGATCCCCTGTCAGCCTT ATTAAACCAATCTGAATTAAGGGTTATCTGTCTGGTTGAACCAC ACTCACCGTTAGTATGGCTGAAAAAGATATCGACATTACTGATC GAAATGCCCGCTTTCACTTCGGTGGAAAAAGCAAGATTATTGAC GGCCCATTTACCCAATCACTGTGCAAATGATATAGATGCAATAA CCTTAAGCCAACGTTACGCATTTAATCCTGAAACCCTGCCATTA ATTTTACAAGAGGCGAAACTTTACCAGCAACAGCGAGATCCTTC AGGTATTTTGCAGCAATGTGATATCCATAAGGCACTCAATTTAC GCGCCCAGCAAAATTTTGGTCAATTGGCACAACGCATTACCCCT | MNTAPVFPDLFQQHNDPHLMFLY SQLERIDLVLQYHFHCISGQKGDL LDEFLLSEEDVINRLDNPLGKPHW LTDDCLTIPHTVTPVASRLTDLITH FELTDFERDVLLLGLLPHFDNRYY HLFALVQGEQQSRLPAFALALELF CHSMLEKQAQQASFLHQAPLVGC QLLSIDSRQKTLSWLQTPFITDSSV YHFLLGHHYIAPALEHCVEWITPT EIGNYPAGLKQALGNVLLSDHDDI RPVILLRGMADSARSHVVANMM ASEQRQTLLVDIASLADNDDNSLF CHIKAILREIRMRGACLLLRNFCLF AEQNKHLLDPLSALLNQSELRVIC LVEPHSPLVWLKKISTLLIEMPAFT SVEKARLLTAHLPNHCANDIDAIT LSQRYAFNPETLPLILQEAKLYQQ QRDPSGILQQCDIHKALNLRAQQ NFGQLAQRITPKRSLKDLLVSDDI AQQIQEILAAIKYREHVLAGGFKD KIGYGTGISALFYGDSGTGKTMA AEVIANYIGVDLIKVDLSTVVNKY IGETEKNLSRIFDLAEQDAGVLFF DEADALFGKRSETKDAQDRHANI EVSYLLQRLENYPGLVILSTNNRS HLDNAFNRRFTFITRFTYPDETIRK KMWQAIWPENIKVAQDVDFDKL AQRTSVTGANIRNIALLSSLFAAE NNHNEVSNKNIETALSRELAKVG RLIF* |
| | AAACGTTCATTAAAAGATTTATTGGTATCTGATGATATTGCTCA ACAGATACAAGAAATACTCGCAGCCATTAAGTATCGGGAACAC GTCCTGGCGGGAGGTTTTAAAGATAAGATTGGTTATGGTACGGG TATCAGCGCCCTGTTTTATGGTGATTCTGGCACCGGAAAAACTA TGGCAGCAGAAGTGATTGCCAATTACATTGGCGTTGACTTAATC AAAGTGGATTTATCTACCGTCGTGAATAAATACATCGGCGAAAC AGAGAAAAACTTATCCCGTATCTTCGATTTGGCAGAACAGGATG CCGGTGTTTTATTCTTTGATGAAGCGGATGCGTTATTTGGTAAAC GCAGTGAAACCAAAGATGCGCAAGACCGACATGCCAATATTGA AGTCTCTTATCTACTGCAACGGCTGGAGAACTATCCGGGGTTGG TTATTTTATCCACCAATAATCGTAGCCATTTGGACAACGCCTTTA ATCGCCGTTTTACCTTTATTACCCGCTTTACTTACCCGGATGAAA CCATCCGCAAGAAAATGTGGCAGGCAATCTGGCCTGAAAATAT AAAAGTCGCCCAGGATGTGGATTTCGATAAATTAGCTCAACGAA CAAGCGTAACAGGGGCGAATATTCGTAATATTGCTTTATTGTCT TCACTGTTTGCCGCAGAGAATAACCATAATGAAGTCAGTAATAA AAATATTGAAACAGCATTATCACGAGAATTAGCCAAAGTAGGC CGTTTAATTTTTTAA (SEQ ID NO: 15; Locus_tag: AB204_RS00520) | (SEQ ID NO: 31; NCBI Sequence ID: WP_047961442.1) |
| XkCis16 | ATGTTAAAAACGCAAACCATTATTGATGTCAATAACGCAATGAA AACTATGCTGGAGAAATATCTGGATAATAAAATTGCAATTCGTT TTGACTTACCTGAATTAGATACCGTGCAATCTGATGCGATGGTG AGTGTCTTTCTGTATGATATCCACGAAGATCTACAGCTTCGTTCG ACAGAATCCAGAGTGTTTAATGCCACTTCAGGGAGATTATTGCC GGGTTGGGTCAATATTAAATGTAATTACCTGATTACCTATTGGG AATCGGCTCAACCAGCAACCGATTCCAACAGCTCAGAATGTCTA CCGGATAATCAGGCAATAAAAGTCATGTCACAAGTCTTGAATGC TCTGATTAATAATCGCCAATTGGCAGGTATTCCCGGTGCTTATAC TCAGGTTGTCCCCCCGAAAGAAAGTTTAAACAGTTTAGGGAATT TTTGGCAATCACTGGGGAATCGCCCCCGACTTTCTTTAAATTACT CAGTGACAATACCGATTAGCCTCACCGATAAGCAGGATAAGGC GACGCCGGTAAGCACCATTTCTTCTACAGTGGAACAAATTGCAT CACTCAGTCAGGAAAAGATTAGCCATACTTTGCGAGAATTATTG ATTACGGAACTACAGATACAACCTGTTACAGAATCAGAAAGTG AAGAGAATATCAGGCTGGCACTGAGCAAAGTTGAACTATTAAC AATGAAAAAGGCTATAACTCCAGACAATTCAGCAGAAATTTCCA TATCCTTGTCTGTATCAGGTATTACCCATCAGAAATATCTACAGG ATATCAATAAAATTTTTGATCGCTGGTTAAATGACAACAAAATT GTTACTACAGTCGATAATTATGGAATCAGGATTAAGGATATAGA AAAAAACCATCTTTTCGGCATTTAA (SEQ ID NO: 16; Locus_tag: AB204_RS00515) | MLKTQTIIDVNNAMKTMLEKYLD NKIAIRFDLPELDTVQSDAMVSVF LYDIHEDLQLRSTESRVFNATSGR LLPGWVNIKCNYLITYWESAQPA TDSNSSECLPDNQAIKVMSQVLN ALINNRQLAGIPGAYTQVVPPKES LNSLGNFWQSLGNRPRLSLNYSV TIPISLTDKQDKATPVSTISSTVEQI ASLSQEKISHTLRELLITELQIQPVT ESESEENIRLALSKVELLTMKKAIT PDNSAEISISLSVSGITHQKYLQDI NKIFDRWLNDNKIVTTVDNYGIRI KDIEKNHLFGI* (SEQ ID NO: 32; NCBI Sequence ID: WP_047961441.1) |

### 2. Construction of Regulatory Factor

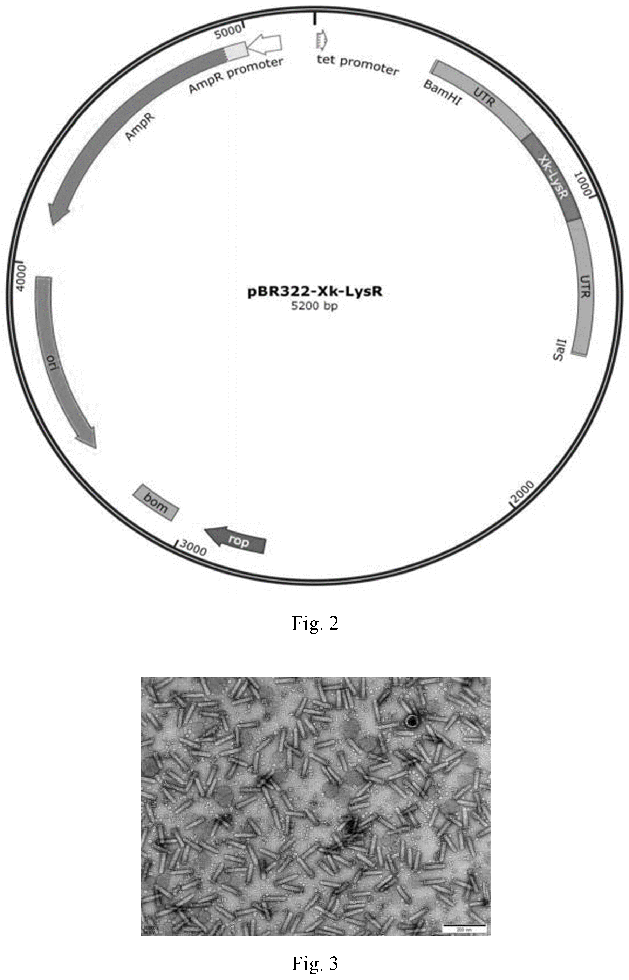

The regulatory factor gene *lysR* is derived from the *Xenorhabdus khoisanae* MCB strain. The gene base sequence and the corresponding expressed protein sequence are shown in Table 2. The complete sequence of Xk-LysR is set forth in SEQ ID NO: 49. The complete sequence of Xk-LysR was ligated into the pBR322 plasmid via the BamHI/Sall restriction sites to obtain the recombinant plasmid pBR322-Xk-LysR comprising the Xk regulatory factor Xk-LysR (Fig. 2).

**Table 2. Xk regulatory factor gene sequence and protein sequence**

| | Base sequence | Protein sequence |
|---|---|---|
| Xk-LysR | GTGTTTATTTCAAAAGACTTAGCAAATTTTATTGTCGTAGCCAAA[?] AATAAATCAATAAATAAGGCAACAAAGGAGCTTTTTATTACACA [?] GTCTCCAATTAGCCGAAGCTTAAAAAAATTGGAGTTCTCATTAG[?] GTGTAAAACTCTTTGTCAGAAAAAACCAAGGATTAGTTCTAACG[?] GAAGAAGGGGAGTGTCTATACAATAAAGTGTTATCAATTTATGA ACAACTTAAAAATATCGAAAATGATTACAAGAATCATAAATCACC CTATTGTGAAATTTATAAAAAACAACATAATGACTTAACATAA (SEQ ID NO: 33; Locus_tag : AB204_RS00505) | VFISKDLANFIVVAKNKSINK ATKELFITQSPISRSLKKLEF SLGVKLFVRKNQGLVLTEE GECLYNKVLSIYEQLKNIEN DYKNHKSPYCEIYKKQHND LT* (SEQ ID NO: 34; Sequence ID: WP_047961439.1) |

### 3. Construction of Protein Expression Strains

The recombinant plasmid pRK404-XkCIS of the XkCIS structural gene constructed in the above steps and the recombinant plasmid pBR322-Xk-LysR comprising the Xk regulatory factor Xk-lysR were transformed into *Escherichia coli* strain (DH10B, Thermo Fisher Scientific, EC0113), and screened in double-antibiotic solid LB medium supplemented with ampicillin and tetracycline.

### II. Protein Extraction and Purification

### 1. Bacterial Culture and Collection

The constructed XkCIS expression strain was picked from a solid LB agar plate, and cultured overnight at 37°C and 220 rpm in 3 mL of triple-antibiotic liquid LB medium supplemented with ampicillin, tetracycline and gentamicin. The culture was then transferred in a ratio of 1:100 to 200 mL of triple-antibiotic liquid LB medium supplemented with ampicillin, tetracycline, and gentamicin, and incubated at 30°C and 220 rpm for 24 hours.

After 24 hours of culture, the bacterial culture was transferred to a 500 mL centrifuge bottle, and centrifuged at 12,000×g for 10 minutes at 4°C. After centrifugation, the supernatant was discarded, and the bacterial pellet was resuspended in 30 mL of phosphate-buffered saline (PBS). The resuspended cells were then transferred to a 50 mL centrifuge tube and centrifuged at 3500×g for 30 minutes at 4°C. After centrifugation, the supernatant was discarded, and the bacterial precipitate was stored at -80°C overnight.

### 2. Bacterial Disruption and Removal of Bacterial Fragments

P buffer was prepared, and the formula is shown in Table 3. 10 mL of pre-cooled P buffer was added to the bacterial pellet collected from every 200 mL of bacterial culture, then incubating at 37°C for 30 minutes to lyse the bacteria, and centrifuging at 4°C, 27000×g for 10 minutes to remove bacterial fragments. The supernatant is retained for subsequent protein purification.

**Table 3. P buffer formulation**

| Components | Concentrations |
|---|---|
| Tris | 25 mM |
| NaCl | 140 mM |
| KCl | 3 mM |
| lysozyme | 200 µgmL-1 |
| DNaseI | 50 µgmL-1 |
| Triton X-100 | 0.004% |
| MgCl₂ | 5 mM |
| protease inhibitor (MCE) | 1% |

### 3. Protein Purification

The supernatant was collected to centrifuge at 4°C, 15000-25000×g for 60 minutes. After centrifugation, the supernatant was discarded, then resuspending the precipitate in 1 mL of pre-cooled PBS. The resuspended solution was transferred to a 1.5 mL centrifuge tube, then centrifuging at 4°C and 14,000 rpm for 10 minutes.

The supernatant was collected again, then centrifuging at 4°C, 15000-25000×g for 60 minutes. After centrifugation, the supernatant was discarded, then resuspending the precipitate in 200 µL of pre-cooled PBS. The resuspended solution was transferred to a 1.5 mL centrifuge tube, then centrifuging at 4°C and 14,000 rpm for 10 minutes.

The supernatant was collected for determining the protein concentration, and stored at 4°C. At this point, the supernatant comprises the XkCIS protein complex, and it is the extracted protein solution.

### 4. Removing Endotoxins

This disclosure uses commercially available endotoxin removal reagents to remove endotoxins from a protein solution. The protein solution and endotoxin removal reagent were pre-cooled, 50 µL of endotoxin removal reagent was added to 500 µL of the protein solution to mix well, then incubating on ice for 10 minutes, incubating in a water bath at 37°C for 20 minutes, and then centrifuging at 37°C and 14000 rpm for 10 minutes. The supernatant was collected.

### III. Results

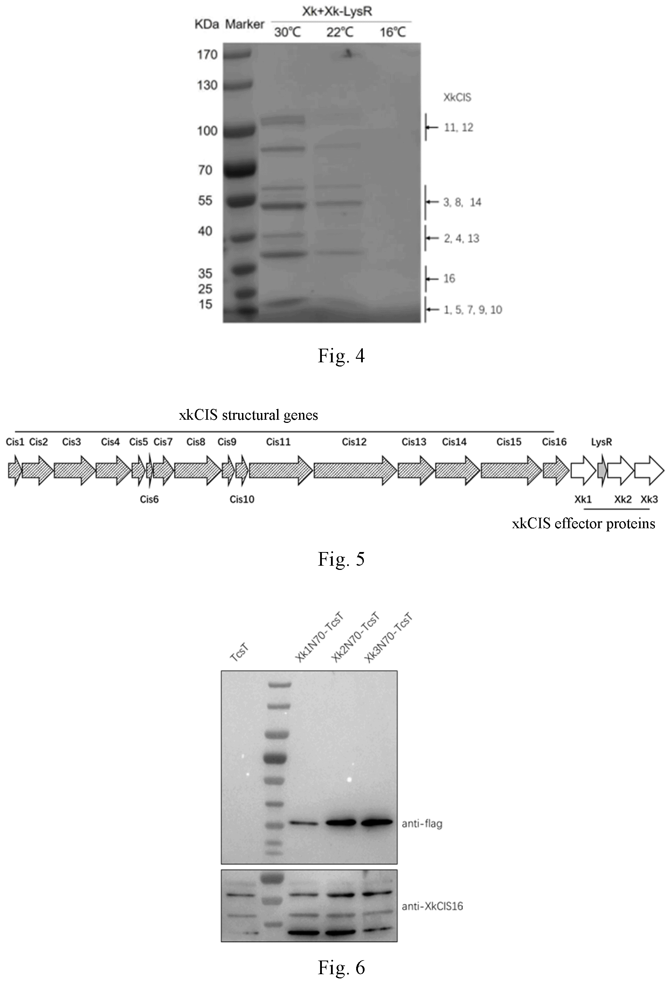

This technical solution allows for the transformation of the XkCIS structural gene and lysR gene into *E. coli,* enabling the expression and assembly of the XkCIS protein complex in bacteria (Figs. 3 and 4), which can be used for subsequent cell or animal experiments.

### Example 2: Downstream Effector Factors of the Xk Gene Cluster Can be Loaded into the Xk Protein Complex, and the N-terminus Has a Signal Peptide Function.

### I. Construction of Cargo Loading Plasmid

The gene cluster of XkCIS structural genes in the *Xenorhabdus khoisanae* MCB strain comprises three effector proteins (XkCIS effectors, Xk1, Xk2 and Xk3) (Fig. 5). The base sequences of the effector protein genes and the corresponding expressed protein sequences are shown in Table 4.

**Table 4. Xk effector protein gene sequences and protein sequences**

| | Base sequences | Protein sequences |
|---|---|---|
| Xk1 | GTGTATAGTTTAAAACAGAAAGAAGAGAAGAAAAATCATTCCC CTCGTCCTACCCAAAATACTGTCAATGGTGATCATGCAATAACA CCAAGAATAGCGGAATTGAGAAAAAACTTTCAGCAGCTTAATT CGCAAACCGTGCGCCCACGTGTTGCTCCTCGTCCATTACATTCA CTTTCGAAGACAGGACCATTTCAACAGGCTACAAAAGCACCTA TTGCCTCAGAGATAATCACTCCTGTCAGCGCAGCTACCAAAACA TTTACCAGTAATACACAAAACCCAAAAGTGACACCAACATCAA CCTCTGCGCCAACAAATGGTGTAAAAAAATCATTTACATTATATC GAGCAGATAACCGAAGTTTTGAAGAGTTACAAAAAAGTAGCCC AGAGGGATTTAAGGCTTGGGTACATCTTGATAGTGAAAAAGCG AGAAAATTTGCCAGCGTATTCTTGGGTAATGACAATGTAGAAAG TTTACCCAAACATATTATTGATGAAATTAATAAATGGAAAAAAG GAGGAACACCTAAGTTGAGTGATCTTTCCACATTTATAAAATAT ACCAAAGATCGCTCAACTGTTTGGGTTTCTACCGCAGTCAATAC TGAAGCAGGTGGACAAAGCTCAGGAGCTCCACTTTATGAAATA TCAATGGAGCTCTATGAATTTGGGGTAGATAAAGGGAAATTGGT TCCATTACCCAATGGAAGAACGGGAAATATGAAACCTTCCATTC ATTTGGATACACAAAATCTCGCAGACGCAACAATAATCGCCCTG AATCATGGGCCAGTTAATGATGCTGAAATGTCATTTTTAACGACT ATTCCGATGAGCAACTTAAAACCCTATAGAAGGTGA (SEQ ID NO: 35; Locus_tag: AB204_RS00510) | VYSLKQKEEKKNHSPRPTQNTV NGDHAITPRIAELRKNFQQLNSQ TVRPRVAPRPLHSLSKTGPFQQAT KAPIASEIITPVSAATKTFTSNTQN PKVTPTSTSAPTNGVKKSFTLYR ADNRSFEELQKSSPEGFKAWVHL DSEKARKFASVFLGNDNVESLPK HIIDEINKWKKGGTPKLSDLSTFI KYTKDRSTVWVSTAVNTEAGGQ SSGAPLYEISMELYEFGVDKGKL VPLPNGRTGNMKPSIHLDTQNLA DATIIALNHGPVNDAEMSFLTTIP MSNLKPYRR* (SEQ ID NO: 38; NCBI Sequence ID: WP_047961440.1) |
| Xk2 | ATGATATACGGTTATGATAACAAGATGGTTTATAGAAAAAGGCA AGAATCGAATACGGCATTGGAAGCTTTGCATCTTGATATACCCG ATACAGCGGAAGTCTCTGCTGCCTATCAGCGCGATTTGTCTTCT | MIYGYDNKMVYRKRQESNTALE ALHLDIPDTAEVSAAYQRDLSSV MTRFQNKTDSILEESKQPSPLKQ |
| | GTCATGACGAGATTCCAGAATAAAACAGACTCAATTCTTGAGG AATCCAAACAACCCTCTCCGCTCAAGCAAAAACCATCAGAGAA AAAAAAATATGCTAAAAAGGCATTAAAAAACTTTGCTGCCCAT GCCGGCTACTCACATATCAACAGCTATCAAGATGAGTTTGTTAA CTTTAAAGATAACAATTATAACTTAGCCCCCGGAAAATTGTTTTG GGGTGTGGAATTAATTCCCCAAAAAACTATCGAAATAAACAGA CCGATAGGACCATGGAGAAATAAACTTTCCTATGATATCGAAGA TATATTTAGAAATAATATGGCAAATGAATATCGGGTAACCAATAC TTTCGAATTTATCAAAGGATTACAGGATATGTATAAAAAAAATG GGCAAACGCTACACCCAATGACCCAACAATTGGTTGAAGAGCA TATTAAACATAACGGGAACATCTTACCAACAATGGCAGGGATTG CCGGTTTGCATGCCGAAGTACAGGCATTAAACCAGTTATTTATT AAAGCAGATGAGGATGCAGGCCAGTCCCCTGAGCCCTTTAGTA TGCGTTATATTAAGGCAATGCTTCAATCATCAATTTTCACCAAAC GATTGACCACAGCGAACGCCGGGCAAGACTTCCCGGCTTGCCA TAACTGCTCGGGTATCATTCAATCACCAGCTAATGTGATTACGG GAACGGTCAGCAGTGCAGGCAGCAATTTTTCTAAGCAGGTCGC AAAACGCGATCGTTCCCAGTCACTAAGTCAATAA (SEQ ID NO: 36; Locus_tag: AB204_RS00495) | KPSEKKKYAKKALKNFAAHAGY SHINSYQDEFVNFKDNNYNLAPG KLFWGVELIPQKTIEINRPIGPWR NKLSYDIEDIFRNNMANEYRVTN TFEFIKGLQDMYKKNGQTLHPM TQQLVEEHIKHNGNILPTMAGIA GLHAEVQALNQLFIKADEDAGQ SPEPFSMRYIKAMLQSSIFTKRLT TANAGQDFPACHNCSGIIQSPANV ITGTVSSAGSNFSKQVAKRDRSQ SLSQ* (SEQ ID NO: 39; NCBI Sequence ID: WP_047961437.1) |
| Xk3 | ATGCCCGTATATGAAAATAAAGAAAAAAGAAAGTATTCAAAAA ATCCTTTACATAATACTTCGACTATTAATCAATTTACTGTTCAAGA TCTGGATTCTGCCTCACAGCAGGAAACAGCAACACCGCTCCTC ACCAATGAATTTAAACAAGGCCACTATGTCAATCACAGTAAGTT ACCTGCTGGTACCAAATTATCTGAGATAGCCATGCTGGGTAGCC ATGATGCGGGAACTTATGCTTATAGTAGGAGAAAATCCGGATTT GCCAGTAGCTTAGGGTCACTGCTTCCTGCGGCATTTAAAACCCA AAATCGAACGTTGAGGCAACAAGCAGAAGCAGGAGCCAGATA TTTCGATATTCGGGTTGCGCAAAATAAAGATGGCTCATTTAGCTT CTTCCATGGCCCCAGCGTTGCAGGCAGTGATGCCGTTTCGGATG TGAAATCGCTCTTGGAATATGCGGCAGGAGATACCAATCACTTT TACTTACTCAAACTCGTATTTAAAGGGGAAAAAGGGCAATCTTC CGCAGCCGCGTCTTCAAATACATTTTTACAAAGTATTCTCGAAG GCCACCAGCAGCGTCTTATTACACTTGATGATACGTCGAGTTTA GGTGAGGCGATGGTCGATTTACTGGATAAAGGAAAAAACATAG GGATCATGGTTGATAGAAAGAAATATGATGGAACAGAACCACAT TGGGGTTATAAAGAAAGTGTTAACACAAAGTGGGCTAACAGAG CGAACGCAGAAGAAACAGCGAATTTCCTTCTTAAATTCCATGA GAATCCAGTACCTGACAAACTCAATATTATGCAGACCAATATCC CTGTTGCTTCCGTAGGGCGTGGGCAATTTACTTCAGGAGTAAAA AGATATTTGTTTGGAAACAGAGACCCATTGATCAAAGCTGTTGC CCAGCTTCCTGCCGGAATAATCAGCGCAGATTACGTAGGCGATT CCGGTAGTGCAACTTCAAAATTTATGGAGACAATTAATCAATAT AACAGGAGCTTAATGGAACGTGATGAAGAGACCAGACTGTAA (SEQ ID NO: 37; Locus_tag: AB204_RS00490) | MPVYENKEKRKYSKNPLHNTSTI NQFTVQDLDSASQQETATPLLTN EFKQGHYVNHSKLPAGTKLSEIA MLGSHDAGTYAYSRRKSGFASSL GSLLPAAFKTQNRTLRQQAEAGA RYFDIRVAQNKDGSFSFFHGPSVA GSDAVSDVKSLLEYAAGDTNHF YLLKLVFKGEKGQSSAAASSNTF LQSILEGHQQRLITLDDTSSLGEA MVDLLDKGKNIGIMVDRKKYDG TEPHWGYKESVNTKWANRANA EETANFLLKFHENPVPDKLNIMQ TNIPVASVGRGQFTSGVKRYLFG NRDPLIKAVAQLPAGIISADYVGD SGSATSKFMETINQYNRSLMERD EETRL* (SEQ ID NO: 40; NCBI Sequence ID: WP_047961436.1) |

To investigate whether the N-terminus of the effector proteins in the XkCIS gene cluster (i.e., the Cis gene cluster) possesses a simple signal peptide that can guide the loading of the protein into the Xk protein complex, taking the TcsT protein (the nucleotide sequence of the gene encoding the TSCT protein is set forth in SEQ ID NO: 48) as an example, three effector protein N-terminal fragments of varying lengths were respectively inserted into pBBRN by double digestion with NdeI/BamHI, then conducting fusion expression with TcsT protein having a flag tag inserted by BamHI/HindIII restriction sites, so as to construct fusion proteins of different lengths to test whether the N-terminus of the effector protein can serve as a signal peptide. The amino acid sequences of amino acids 1-130 of the N-terminal signal peptides of the three effector proteins Xk1, Xk2, and Xk3 are shown in Table 5. Their sequences are SEQ ID NOs: 44, 45, and 46, respectively. The amino acids 1-30 (respectively represented as Xk1N30 (SEQ ID NO: 54), Xk2N30 (SEQ ID NO: 55), and Xk3N30 (SEQ ID NO: 56)), the amino acids 1-50 (respectively represented as Xk1N50 (SEQ ID NO: 57), Xk2N50 (SEQ ID NO: 58), and Xk3N50 (SEQ ID NO: 59)), the amino acids 1-70 (respectively represented as Xk1N70 (SEQ ID NO: 60), Xk2N70 (SEQ ID NO: 61), and Xk3N70 (SEQ ID NO: 62)), and the amino acids 1-90 (respectively represented as Xk1N90 (SEQ ID NO: 63), Xk2N90 (SEQ ID NO: 64), and Xk3N90 (SEQ ID NO: 65)), the amino acids 1-110 (respectively represented as Xk1N110 (SEQ ID NO: 66), Xk2N110 (SEQ ID NO: 67), and Xk3N110 (SEQ ID NO: 68)), and the amino acids 1-130 (respectively represented as Xk1N130, Xk2N130 and Xk3N130) of SEQ ID NO: 38, 39, or 40 were respectively selected to conduct fusion expression with TcsT. Meanwhile, a TcsT without a signal peptide was used as a negative control to test the guiding role of the signal peptide in TcsT loading.

**Table 5. Xk signal peptide gene sequences and protein sequences**

| | Base sequences | Protein sequences |
|---|---|---|
| Xk1-signal peptide | GTGTATAGTTTAAAACAGAAAGAAGAGAAGAAAAATCATT CCCCTCGTCCTACCCAAAATACTGTCAATGGTGATCATGC AATAACACCAAGAATAGCGGAATTGAGAAAAAACTTTCAG CAGCTTAATTCGCAAACCGTGCGCCCACGTGTTGCTCCT CGTCCATTACATTCACTTTCGAAGACAGGACCATTTCAAC AGGCTACAAAAGCACCTATTGCCTCAGAGATAATCACTCC TGTCAGCGCAGCTACCAAAACATTTACCAGTAATACACAA AACCCAAAAGTGACACCAACATCAACCTCTGCGCCAACA AATGGTGTAAAAAAATCATTTACATTATATCGAGCAGATAA CCGAAGTTTTGAAGAGTTACAAAAAAGTAGC (SEQ ID NO: 41) | VYSLKQKEEKKNHSPRPTQ NTVNGDHAITPRIAELRKNF QQLNSQTVRPRVAPRPLHS LSKTGPFQQATKAPIASEIIT PVSAATKTFTSNTQNPKVTP TSTSAPTNGVKKSFTLYRAD NRSFEELQKSS (SEQ ID NO: 44) |
| Xk2-signal peptide | ATGATATACGGTTATGATAACAAGATGGTTTATAGAAAAAG GCAAGAATCGAATACGGCATTGGAAGCTTTGCATCTTGAT | MIYGYDNKMVYRKRQESN TALEALHLDIPDTAEVSAAY |
| | ATACCCGATACAGCGGAAGTCTCTGCTGCCTATCAGCGC GATTTGTCTTCTGTCATGACGAGATTCCAGAATAAAACAG ACTCAATTCTTGAGGAATCCAAACAACCCTCTCCGCTCA AGCAAAAACCATCAGAGAAAAAAAAATATGCTAAAAAGGC ATTAAAAAACTTTGCTGCCCATGCCGGCTACTCACATATC AACAGCTATCAAGATGAGTTTGTTAACTTTAAAGATAACAA TTATAACTTAGCCCCCGGAAAATTGTTTTGGGGTGTGGAA TTAATTCCCC AAAAAACTATCGAAATAAAC (SEQ ID NO: 42) | QRDLSSVMTRFQNKTDSIL EESKQPSPLKQKPSEKKK YAKKALKNFAAHAGYSHIN SYQDEFVNFKDNNYNLAP GKLFWGVELIPQKTIEIN (SEQ ID NO: 45) |
| Xk3-signal peptide peptide | ATGCCCGTATATGAAAATAAAGAAAAAAGAAAGTATTCAA AAAATCCTTTACATAATACTTCGACTATTAATCAATTTACT GTTCAAGATCTGGATTCTGCCTCACAGCAGGAAACAGC AACACCGCTCCTCACCAATGAATTTAAACAAGGCCACTA TGTCAATCACAGTAAGTTACCTGCTGGTACCAAATTATC TGAGATAGCCATGCTGGGTAGCCATGATGCGGGAACTT ATGCTTATAGTAGGAGAAAATCCGGATTTGCCAGTAGCT TAGGGTCACTGCTTCCTGCGGCATTTAAAACCCAAAAT CGAACGTTGAGGCAACAAGCAGAAGCAGGAGCCAGAT ATTTCGATATTCGGGTTGCGCAAAATAAAGATGGCTCAT TT (SEQ ID NO: 43) | MPVYENKEKRKYSKNPLH NTSTINQFTVQDLDSASQQ ETATPLLTNEFKQGHYVNH SKLPAGTKLSEIAMLGSHD AGTYAYSRRKSGFASSLGS LLPAAFKTQNRTLRQQAEA GARYFDIRVAQNKDGSF (SEQ ID NO: 46) |

### II. Construction of Protein Expression Strains

The obtained pBBRN plasmid was transformed into an *Escherichia coli* strain comprising the XkCIS structural gene recombinant plasmid and the Xk-lysR recombinant plasmid, then selected on a triple-antibiotic solid LB medium supplemented with ampicillin, tetracycline and gentamicin.

### III. Methods of Protein Expression and Purification are the Same as Those in Example 1.

### IV. Results

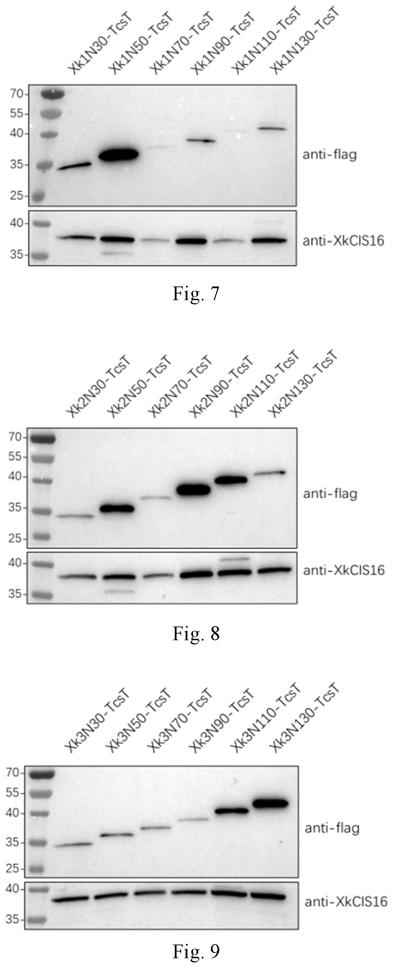

The signal peptide sequence is derived from the N-terminal sequence of the XkCIS effector protein, comprising the N-terminal 1-130 amino acids and a truncated sequence thereof. As shown in Fig. 6, without a signal peptide, the TcsT protein cannot be loaded into the Xk protein complex, while with presence of Xk1, 2, and 3 signal peptides, the TcsT protein is loaded into the Xk protein complex. Figs. 7-9 show that the N-terminus of effector proteins Xk1, Xk2, and Xk3 of different lengths can guide the loading of TcsT protein into the Xk protein complex.

### Example 3: Signal Peptide Guides Different Proteins to Load into the Xk Protein Complex

To verify whether the N-terminal signal peptides of the Xk protein complex effector proteins Xk1, Xk2, and Xk3 can guide the loading of other different proteins into the Xk protein complex, the inventors constructed *E. coli,* which co-expressing the Xk protein complex structural gene and Xk-lysR, along with various cargo proteins fused to the signal peptides. Specifically, the N-terminal 70 amino acids of Xk1, Xk2 and Xk3 (respectively the amino acids 1-70 of the sequences set forth in SEQ ID NOs: 44-46) were fused to the N-termini of BlaM, Cre, and RluC, each bearing a C-terminal FLAG tag, as well as to GFP without a C-terminal tag. The detailed methodology is as follows.

### I. Construction of Recombinant Plasmids and Protein Expression Strains

The N-terminal 210 bp base sequences of *Xk1, Xk2,* and *Xk3* were respectively inserted into the NdeI/BamHI restriction sites, followed by the insertion of BlaM, Cre, and RluC proteins after them, respectively, and a FLAG tag was introduced at the C-terminus and ligated into the pBBRN plasmid (published in the literature: Jiang et al., N-terminal signal peptides facilitate the engineering of PVC complex as a potent protein delivery system. Sci Adv. 2022 Apr 29;8(17):eabm2343, which is incorporated herein by reference). The GFP protein is also inserted into the NdeI/BamHI restriction sites, but there is no FLAG tag at the C-terminus.

The recombinant plasmid for expressing the structural genes and the recombinant plasmid for expressing the regulatory factors were co-transformed into *Escherichia coli,* then selected on solid LB medium supplemented with the corresponding double antibiotics to obtain a protein-expressing strain.

### II. Methods of Protein Expression and Purification are the Same as Those in Example 1.

### III. Results

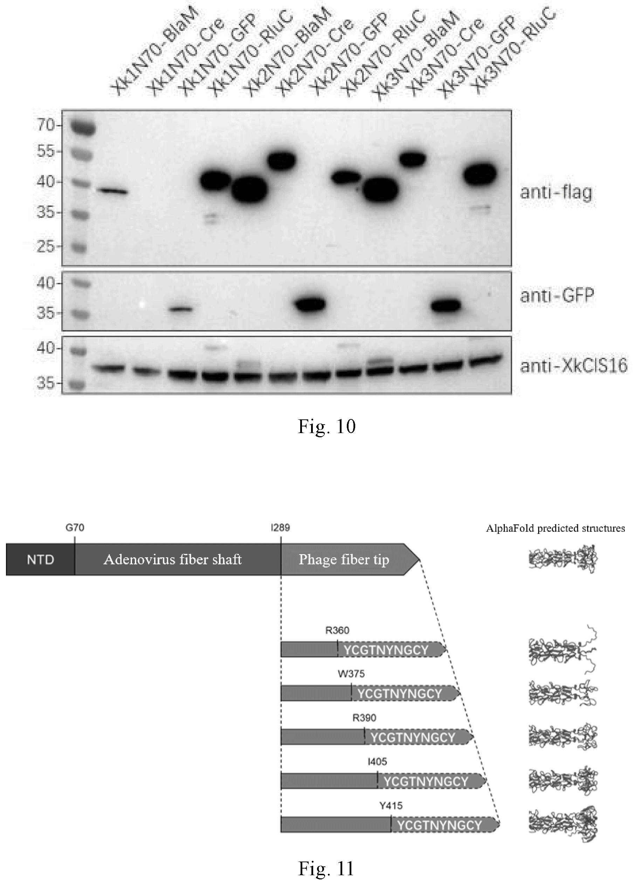

As shown in Fig. 10, all three signal peptides can guide the loading of different proteins, such as BlaM (the nucleotide sequence encoding the gene is set forth in SEQ ID NO: 50), Cre (the nucleotide sequence encoding the gene is set forth in SEQ ID NO: 51), GFP, and RluC (the nucleotide sequence encoding the gene is set forth in SEQ ID NO: 52), into the XkCIS protein complex.

Through the above technical solution, the effector protein (comprising virulence factors or reporter proteins of Xk itself, cellular or bacterial virulence proteins, transcription factors, regulatory proteins (e.g., signaling pathway regulatory proteins), hormone or hormone regulatory molecules, structural proteins, transport proteins, antimicrobial peptides, enzymes involved in cell metabolism, gene editing proteins, antigens or immunogens, medicament-active proteins, and other polypeptides or proteins) that conducts fusion expression of signal peptide is loaded into the XkCIS protein complex under the guidance of the signal peptide.

### Example 4: Modifying XkCIS13 to Enable the Xk Protein Complex to Specifically Target a Cell

To enable the Xk protein complex to specifically target a cell to delivery protein, the inventors replaced the recognition region of XkCIS13 with other recognition proteins (Fig. 11), and co-expressed the modified XkCIS13 and other Xk protein complex structural genes, as well as Xk-lysR and the fusion expression TcsT having an N-terminal signal peptide and a C-terminal flag tag, in *Escherichia coli.* The M1 peptide fragment used in this embodiment has been previously reported to bind to the surface receptor of human retinoblastoma cells Y79.

The detailed methodology is as follows:

### I. Construction of Modified XkCIS13 Structural Gene Plasmid

By seamless cloning, the M1 sequence (SEQ ID NO: 69) was inserted after amino acids 360, 375, 390, 405 and 415 of **XkCIS13** to replace the native recognition region (the receptor binding domain) of XkCIS13, and the modified XkCIS structural gene plasmid was constructed, using the same method as in Example 1.

### II. Construction of Protein Expression Strains

The modified XkCIS structural gene plasmid was transferred into an *Escherichia coli* strain containing a recombinant plasmid comprising the signal peptide-TesT protein and Xk-lysR recombinant plasmid, then selected on triple-antibiotic solid LB medium supplemented with ampicillin, tetracycline and gentamicin.

### III. Protein Expression and Purification

The method is the same as in Example 1.

### IV. Cell Experiments

Y79 cells were treated with modified XkCIS after extraction and purification. Y79 cells were respectively treated with final concentrations of 1 mg/mL, 0.7 mg/mL, 0.4 mg/mL and 0.1 mg/mL for 36 hours. Cell viability was assessed using the CCK-8 assay.

### I. Results

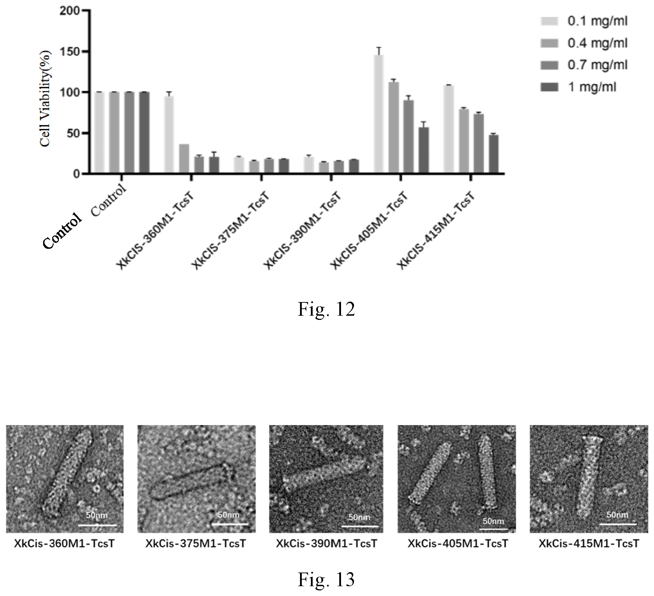

Following a 36-hour treatment with modified XkCIS protein complexes carrying the TcsT toxin (Xk-360M1-XK3N70-TcsT, Xk-375M1-XK3N70-TcsT, Xk-390M1-XK3N70-TcsT, Xk-405 M1-XK3N70-TcsT, and Xk-415M1-XK3N70-TcsT), pronounced cell death was observed in Y79 cells (Fig. 12). Meanwhile, the survival rate of the cells was tested by the CCK-8 assay, revealing that the XkCIS protein complexes with different modified sites exhibit varying cell-killing efficiencies (Fig. 12). Direct observation by transmission electron microscope revealed that uniform modified XkCIS particles were obtained after purification and extraction (Fig. 13).

The XkCIS 13 protein has a total length of 419 amino acids. The targeting recognition region of XkCIS is located after amino acid 355. The best-performing modified region in this targeting recognition region is located between amino acids 360 and 405. The modified XkCIS protein complex can target and recognize a cell and inject the effector proteins it carries into the cell.

Some of the sequences used herein:
Complete sequence of the XkCIS gene cluster *(Cis* gene cluster) (the underlined portions are the coding regions, nucleotides 1-7714 are set forth in SEQ ID NO: 47, nucleotides 7715-19853 are set forth in SEQ ID NO: 53):

ATTCCTGGATTGCGCCAAATTATGTCTTAAGAAATTTCTATAACCCCCAATTATCACATCTACACCTCCCTAT
TATTTATCTATTAACAACTTTAACATTCGATCTTTATTTAATCTTTATTTCACTATTAATATTTTACATTTTATCTA
TTAAATCATTTTTAACACGCATTAATTAAATGTCATTAAGCAATTCTTAAACAACTTCTTATTTTAAGAAAA
ATAAAATTGCATAAAAAGCAATTTGCATTATTGGCAATGTACTCATTATAAATTTTTCGGCACGATAAATATC
GATATAAGCCTGATACAGCCACATACAATTTTTTCAGAGTGAAGTTTTTTTCAGAATGAAGTTTACTTTCA
TTTTGTGGCGGTAGCTTTATCTGAGCATATATTTTCTGCATCCTATTTAATCAATTACAACCATTTTTATTTA
AAAAAGAGGCTACTATGTCTACAAGCACATCTCAGATTGCTGTTGAATACCCTATTCCTTCTTATCGTTTCG
TTGTTTCCGTCGGAGATGAACATATTCCATTTAATAGTGTTTCAGGGCTGGATATTAATTATGAAACCATTG
AATACCGTGATGGCGTGGGTAGTTGGTTTAAAATGCCGGGTCAGCGTCAGCCGATTAATATTACCCTGCG
TAAAGGTGTTTTTCCGGGCAAAACTGAATTATTTGACTGGATTAATTCTATCCAACTTAATCAGGTTGAG
AAAAAAGATATTTCCATTAGTTTAACCAATGACGCAGGCACTGAATTATTAATGACCTGGAATGTTGCTAA
TGCTTTCCCTACCTCATTGACGTCTCCTTCCTTTGATGCCACCAGCAATGATATTGCCGTTCAGGAAATTA
CCCTGACAGCCGATCGCGTCACCATGCAGGCTGTTTGAGGCATTAATAGTTAATCATTTCGTATAAAGGAT
TTTTTTATGACAACCGTTACCAGTTATCCTGGCGTTTATATTGAAGAATTAAATAGCCTGGCATTATCCGTAT
CAAATAGTGCCACCGCCGTTCCTGTTTTTGCAGTGAACAAAGAAAACAAACAGATTAATGAAGAGACT
GCACACCGAATTAATTCCTGGATGGATTATCTTAATCTGGTAGGCAACTTTGACAATGAGGATAAATTGT
CAGTTTCTCTGCGGGCTTATTTTACCAATGGCGGGGGTTATTGTTATCTCATCAAAACCCCAAATTTAGAA
AAACTTATTCCAACCTTAGATGATGTCACTTTGCTGGTTGCGGCAGGTGAAGATATTAAAACAGCCGCG
GCAACTTTATGTCAGCCGGGAAAAGGGTTATTTGCCATTTTTGATGGCCCTGAAGAAGCGTTGACCATC
GCGAATGCAGAAACAGTCAAGAACAAATATGACGCCACCGCGTTCGCCGCAGTTTACTACCCGTGGCT
GAAAACGGATTGGGCAAAAATCACCATTCCACCGAGTGCGGTTATGGCAGGCGTTTACGCCTCCGTCG

ATCTCTCCCGTGGCGTGTGGAAAGCTCCCGCCAATATCGCATTGAAAGGCGGACTGGAACCTGCATTTT
TGATCACGGATGAAGTTCAGAGTGCATATAACTCCGGTCGTGCCATCAATATGATTCGGAATTTCAGCAA
TACCGGTGCCACCGTTTGGGGAGCAAGGACGCTGGAAGATAATGACAACTGGCGCTATGTCCCTGTGC
GTCGCTTATTCAATACCGTCGAGCGGGATGTTAAGCGCGCTATGAGCTTTGCCATGTTTGAACCTAACAG
CCAACCAACGTGGGAACGTGTCAGGGCGGCAATCAGCAATTACCTTCATAGCCTGTGGCAACAAGGG
GGATTGGCTGGCAGCAAAGAAGAAGATGCCTATTTCGTGCAGATTGGCAAAGGAATTACGATGACGCC
AGACCAAATTAATCAGGGACAAATGATAGTAAAAATCGGCCTGGCGGCCGTTCGACCGGCAGAATTTAT
CATCCTCCAGTTTACGCAAGATGTAGAACAGCGTTAATCACATTAATTGATGAGGAATTATCATGCCAGCT
AATCTAAAAACGCCCGGCGTTTATATTGAAGAAGATGCTTCCCTTGCATTATCGGTCAGCAACAGTCCCA
CTGCCGTGCCTGTTTTTATTGGTAAGTTCACCCCAAAAACTGCGGAGCCAACCCAAACCTGTATTCGTA
TCAGCAATTGGCTTGAATTTACTTCCTCTTTTTCTTTGGCACCAGTGACAGAAATTGTCATCCGCTCTAG
TCTTGAAGCCTCTTCTGATGGCGCAACAAAAAAAGAACAGGCCAAAAAAGAAAAAGTCGAAAATAA
AGAGACTGCAACCCAAGAAACTCCCAAAGTTCAAACTCTCTCGGCAGCCGAAGAAGAACCCAAAAA
ATGGATTCACAATATTGAGAGAGTCAATCTATCTCAAGCCGTAGAAGCCTTGAAGCTCTATTTTCAAAAT
GGAGGCGGGGCTTGTTATATCTACCCATTAAACAATATTAATGGCGAGTCGGCTCTGGACCCCATTCCTAA
GGTGATAGAACAAAAAGGGGATATCACTCTGTTGGTTTGTCCAGAAGAAGATCAGGGTTATAAAAACA
GAGTCTACAGTGCAGTCAGCCCCCTATTGAATAGTGATAACAAAAAAGTGGGCTATTTCCTGATTGCAGA
TAGCCTTGATGGTAAATCGGTGTCTGGCATGGCAAATAGCACCAAGATCGCCGCCTATTATCCTCAGTTG
GAAACAAACCTGAAATTCTCCACCTTGCCCGACGATCAGAACATTGTTATCAGTGGTTATGAGAACGCA
TCAGGCAAGGACAAGCTGAAAACTCTGGATAAACTTAGGGAAACAGATAAAGCGCTGGCACAAGAAA
TTGATATCAGATTGCAAGAAATACAACAAGATAATCTCACCCTTTCACCCAGTGCTGCCATCGCAGGAAT
TTATTGCCAAACTGATAGCCAGCGTGGCGTCTGGAAAGCTCCCGCCAACGTTGCACTCAGCGGAATCA
AAGGTCTGACTGATAAAGTCGATGACGAACGGCAAGGCACCATGAATGAGAATGGCATCAATGTCATC
CGTTCATTTACCGGCCGTGGCTTGATGGTTTGGGGGGCGCGTACTTATGCTCAGAACGGTGAGATCAAC
TGGCGTTACATTCCTGTCCGTCGCCTGTTCAATTCCGTTGAACGTGATATCCGTCAGGCACTGCGTGCC
GTGTTATTTGAAACCAATAATCAACCGACATGGATGCGCGCCAAGGCTGCCGTTGACCAATATCTTTATG
CCCTCTGGCAAAAAAATGCCCTGATGGGGAACAGCCCGGAAGAGGCTTATTTTGTCCATATCGGTCAGG
ATATCACCATGTCTGATGCAGATATCAAACAAGGCAAAATGATCATGAAAGTAGGTCTGGCCGCAGTGC
GACCGGCTGAATTTATCGTCCTGCAATTTACGCAGGATGTCGCTCAGTAATCTCCGTGATTAAACACCGG

GCACTGTATTAACAGTGCCTATCTCACCCAATTAAAGGAGATGATGATGGAAAGAATTCAACCGGGCGT
GACCTTGACAGAAAGTATAATTACCACAGGCCAGCAAGAGATACCCAGCGCAGTGCCGGTATTTATTGG
CTATACCGTCCGCAATCAACAACAAGCAAAAACCGCAGTTCGTATCGACAATCTGGCGGAATATACCCA
GCGATTTGGCGACGATCATCTGATGGCATTGGCGGTAAGGCACTATTTCGATAATGGCGGACAACGGGC
TTTTGTTTTGCCACTGTCAGGAAATATGCCCTCAGCAAACATGACCCAAGCGGAAGCCGAAGCACTGAT
GGCGGCATTAAGCTCTGACATGGTGCAAGAAGCCATTGGTGGGGAAACGCAGATCACCCTGATTTTGG
CACCGGATATCGCCCGACTGAATGACAATGTGACTGATGAGCAACAAACTTTAGTCAATTTGTGGTCAC
AGGGTTGGAGTACACTGCTGCAACTAAGCCAGATCCGCACGAATCTCTTTGTGCTGCTGGATGCGCCG
GATGAGGTTGAACAGGCACGGCAATGTCTGGAAGGACTCTCGTCAGAGTATCGCCAATGGGGAGCCG
CTTATTGGCCTCGTCTGGAAACCACTTATCAGGCTACATCCAGTTCCAAAAATATTATGCTCTCCCCCACT
GCCGCTATCGCAGCACTTATCCAACACACGGATAGCGAAGTTGGTGTATGGAAAGCGCCAGCCAATATT
GCCCTGTCTCAGGTTATCCGGCCGGTTAAGTCGTATCTTCAAGGGAGTGTACTGTTTGACACCAATGGC
ACTTCGCTCAATGTGATCCGCACTTTCCCCGGCAAAGGCATACGCGTATGGGGATGCCGCACACTGGAA
AACACAGATAACACGCAGTGGCGTTACCTACAAACACGACGTCTGGTTTCTTATGTCACCGTACATTTG
GCGCAATTAGCCCGTATGTACATCTTTGAACCTAATAACGAACTGACGTGGATGAAATGCAAGGGACAA
AGTTACAACTGGCTACGGCAACTGTGGTTACAGGGTGGCTTGTACGGCTCACGGGAAGATGAGGCATT
TACTGTGCTGTTAGGCGTCAATGAAACGATGACCGAAGAGGATATTCGTGCCGGGAAAATGATAATGAA
AGTCGCCTTAGCCGTATTGTCTCCCGCGGAATTTATCGAGATCAGTTTGGTATTTGATACCCAAACGGGC
GCGCTTTTGTCTTAAAAAGGAAAGAGCACAATGAACGATTATTACACCCCCGTGGTATCCCACCGTTTTA
TGGCGAGCTTTATTTTTAATGGCATTCCTGATCCACTGGATATCCGTTTTCAACGTATTTCCGGCCTGAAC
CGGGAACTTCAGGTCACCCAGCACAGCCAAGGCGGTGAAAACGCCCGCAATAACTATCTGGCTGAAA
AAATCCAACACGGCACATTAACGCTGGAGCGCGGTGTGATGACGGTTTCTCCTTTGACCTGGATGTTTG
ACCGTGTATTGCGTGGAGAAAATATCGCCTATGCGGATGTGGTGATTATGCTGTTAAACGAAAATTCGCT
GCCCTTGTCCAGTTGGACGCTGAGCAATGCCTTACCGGTACGCTGGCAGACCAGCGATTTTGACGCGA
ACAGTAATGCCATTCTGGTGAATACCCTTGAATTGCGTTATCAGGATATGTGCTGGCTGGGAGTCAAAGT
ATGACCGTCGAAATCAGAGAATTAATTATTCAGGCAAAAGTGGTTTCATCATCACCAATACTCCCAACAC
AAGCAACTGAATCACATCAACAAAGCCATTCTTTGATTCAGGAAAGTCTGGATGAGGCAGCTTTAGTTG
AAAAAATTAAACGCGAAGTGCTGGCTGCATTACGCGATGAGGAAGGATGGCGCCCATGAGTTTAATTG
AACGTGGTTTAGCGAAGCTGACGATTAACGCTTATAAAGACAGGGAAGGAAAAATACGGGCTGGCACC

ATTCAGGCCATGTATAACCCTGATTCCCTGCAACTGGATTACCAAACGGATTACCAACAATCCCAGGCAA
TTAATAGCGAAAAGCAGAGCAGCATTTATGTACAGGCCAAGCCTGCCGGGTTATCCCTTGAGCTGATTTT
TGACGCCACAATGCCGGGCAACAAAACCCCTGTTGAAGCTCAGTTAATGCAGCTCAAGCAGTTGTGC
AGCGTGGATGCCACCAGTAACGAAACGCGGTTCCTGCAAATCAAATGGGGAAAAATGCGTTGGGAAA
ACCGCGGCTATTTTGCTGGCAGGGCGAAAGGTTTGTCTGTGAATTACACCTTGTTTGATCGTGATGCCA
CGCCATTGCGGGCGCGGGTCATCCTGACATTGGTGGCGGATGAAAGTCTGGTCTTACAGGAGACAGAG
CAAAACCTGAAATCGCCGGCGAAAATTGCCTTACGGGTACAGGATGGTGTTTCGCTGGCCCTGATGGC
GGCAAGCACCGCATCAACGCTGTCTGGTGGTGTTGATTACCTGACGCTGGCCTGGCAAAACGGTCTGG
ATAATCTTAACGGATTTGTACCGGGTGAGATATTGCAGGCCACCAGAGGAGATGCCTGATGAGCCATCA
ACTGAAAATCATTGCCGATGGCAAGGCACTGTCTTTGCTGGCCGCCGTTGATGTCGATACTTACTGCCG
CGTTAACAGCATCCCTTCCGCAACGCTGAAACTCAGCCTGCCGGACAGCCCTCTTTCCTCTTTCAGCC
AAACGGATGTACAGGCAGAACTGGCCCATTGTCAGGCAGGGAAAACCCTGCGTCTGGAATTGACTGA
CAGCGGCAGAAAATCAGTGCTGTTTAACGGCCTGATTACCCGTAACTCTTTGAAGATAAAAAACAAGC
AATTATTGCTTACTTTGGTTGTCCGACATCGGTTGCAACTGATGGCAGATACTCAGCACTCACAACTGTT
TAAGGCCAGTAATGAAAAAACCATTCTGAACACCTTATTGAATCAGGCGGGTATCAAGGCTCGTTTTGA
GCGGATAGACGCACTGGATCAAAAACATGAACAGATGGTGCAATTTCGCTGTTCAGACTGGCGTTTTC
TGTTGTGCCGACTATCGGCAACCGGCGTATGGCTGTTACCCACGACAGAAGGTGCTCAGCTTGTCCAG
CCTAACACCCTGAAATCCAACCCAACCCATACCCTGAAAAGCCGGGGAGATGAGAAAAAAGATATCAT
CGTCAAGGATGCCAACTGGCAGTTTGATAACCATCTTAACCCTGCTTCGCTGGAAGTCAGCGGCTGGGA
TATCAGTAAACAGCAAGAATTATCAGGTGGTCGCCACGGCAGTGTTGCCATGGGCAAAGCCGCACTTT
CTCCTGACAGGCTGGCGTCTTTAAATAAAACAAGCTGGGACATTCGCTACAGCAGCCCGTTAACCACC
CAAGAGAGTGGCTATCTGGCACAGGGACTGTTGTTGAATCAGCGCATTTCTGGTGTTACAGGCGAATT
CCTGCTTAAAGGTGATGGACGTTATCAGTTAGGGGACAACATTCAATTAACGGGATTTGGTTCACAGCT
GGATGGAACAGCAAGCATTACTGCGGTTCGCCATCGTTTTAACCGGCGAATTGATTGGGAAACCACTTT
AAGCATTGGCTTACCACAGGAATATCTGCCGATGCTACCCGATGCCACCGGACTCCATATTGCGACAGTC
GCGAAGTATCAGCAGGACAAGGCAGCCCTGAACCGCATTCCGATTATTCTGCCGGTACTGAATCGTCCG
AATGAATTCTTGTGGGCCAGATTAGGGAAACCTTACGCCAGCCACGAAAGTGGTTTCTGTTTTTACCC
AGAACCCGGTGACGAAGTGATTGTCGGCTTTTTTGAAAATGATCCTCGTTATCCGGTGATTCTGGGGGC
AATGCACAACCCGAAAAATAAAGCGCCTTTAGAACCCACCCAGGATAATCAGGAAAAAGTCTTGGTGG

TGAAAAAAGGAGAAATCCAACAACAAATCATGATTAATCGCAAAGAGAAAACTATTCAAATGAGTGCA
GGAGAAAACCAGATAATGCTCCAGCAGGATAAAGATATTTCGCTGTCAACGAAAAAAGAATTAACACT
GAAAGCGCAGACCATGAATGCCGAAATGGAGAAATCACTCAATATTTCTGGCAAAAACAGTGTTGAGA
TCAAAGGCAGCAAAATTAATCTTACCCAATGAAAGGTGAACAATGGAAAACCAAATACTGACACAGCT
TTATGGTCGTGGCTGGGCTTTTCCTCCCGTCTTTTCCCTTGAAAAGGGGGTGGAAATGGCTGAGGGGA
CAGAAGATGTGCGCCAAAGTTTGCATATTCTGTTCAGTACTGAGCCGGGGGAACGCCTGATGCGCGAA
AATTACGGCTGTGGATTAAATGATTTTATGTTTGAAAATATCCGCAACGAACTGTTTGCTGAAATTGAATC
CCATATCCATGACAGCGTATTACGTTATGAATCACGGGCAGATATTACCGATATCCAAGTTCGTCAGTCGCC
AAATAGCAAAAATACCTTGCAAGTGCAGGTGATGTATCGCCTGCGGGGCAGCGATATCAATCAGCAAAT
TCAAGGAACACTGGCGCTGGGTGAAGGCCAGGGAATGGAGGTGGTATGAGTGAACCCATTGTAACTG
ACGGCGACACATTACAGTTTGAGCCCCAATTTGAGAGTCGAAAGGTGAACATTCCCGGATTGATTAAAA
TCAATGGAACAGGTCATGCACAGGTCAACGGCAAAAAAGTTTGCATTTTGGGAGACGAGAAACGGGTT
ACTGTTTCTGCAACTTATACCACAACAACTCATATGACACCGGGAACTGGCACCATTACTATCACGGCGT
TAGATAGCAGCCAGCAAGCCCTTCAATGTACCAGTGATGCTGCTTTAATTATCAAAGGGAAAAAATTTAC
GGCCATATTTACGCCTCAATCACCAGCCATAAATGGTAATGTGAATCCACCTCAACCTGATCCTAATTACC
CAAGCCCTTCATCAGGCAAAGGCAGTTTTATCACCCAACAAAACTTTGCCACTGTGAATTAAGGCATTG
GCTGAATTAAATAGAAATTAAAGGGTGTGAATAAGTATTTATTCACAGAGGAATCGCTGTGCCACACAGG
TGATATTATGGAATTAACTGAGTTAAATAAAAAATTGTCAGATTTGGTGCCGATGAGCGATTTTAAATTAG
ATAGCCGAACCAGTTTGCAATTACTCCAATATATTGAGGAGTACACTAAAATAATTCCCTTTAATCAAACC
GATAAGCATTGGAATGATTTTTTCTTTAAGAAAGATAGTACGCCAGAGAAACTGGCCGAATTATATCAAG
GGAAAATCGAAGCCAATGGGAATTTATTACCTCATCAAGCTTTTCTACTAGCGGTATTACGATTACTGGAA
ACACCAATATCCTTATTGAATACCTTACCGGCGGCGCATCGCAATCTCTATTATCGGGCGCTTTTGGGGCT
ATCTCCCCGTCCGGCTCGACCTGATCAAGTTGCCTTATCTGTAGAACTGAATACCACAGTCACAGAACA
ACTACTCACTAAAGGCACTTTATTTGAAGCAGGGCAAGATGAACACGGCAATGCCTTGCAATATGCGCT
GGATACCAGTTTGCTGGCAAACCGTGGCTATATTAGTGAATTGCGCTGGCTCCGTAAAAAAGAACAAC
AGCAATGGATCACCGCGACTCCCTGGAATTCACAGGCACAATTATCATTGCCATCTGGTGGAATACGGT
TATTCAGTGAAACTGATTTGGATAAATCGGTACTGGGCGGGATGTTGATCACTTCCAAACATCTGGCGAT
GGAAGAAGGAGCAAGAGAGATAACGATTACCTTTGATAAGGACATGGATAGCCAGAATTTGGTGGCAC
AAATCAGAAGTGGTAATCAATGGCTAACTTTACAAGTTGAGAAAAATACAGCTGAGAGAAATAAAAAA

GAAGTCATAATGATGCTGGCAGATAAAGAGCCAGCAACCCGTGCACCAGAGGATCTGGATAACCTGATT
TTCAGTGAGCCAGTACTCAGGCTACAGGGAAAAAACAGCCAACCACTGCCTACAGTCACCGCACTAA
AGGTGCAAAAAACAACTATAGATAAAGCTTCATTCAAGACATTTCACTTAACCCCCTTTGGTTATAGCCC
TGAAATTGAACCATTGGAAGAAAACCCCGCATTATACCTTGGTATCACTGACGTCAAGCCGGGACAAAC
CCTATCGCTGTATTGGAAATTAAAATCGCCACAGCAACCCGAAAACGTTTTCTGGTATTACCTGAATAATA
AGAATCAATGGGTTGCATTAGATGCGTGGGTCAAAGATGAAACCAAATATCTGTATCAAGATGGCATCTG
GCAAGTTGTATTGCCTGCGGATGCTTCTGCTTCTGCTGAAGAAATTACCCGGATGCCAACAGGGCGCTA
TTGGTTAAAGGCAGTCATGGTTATCCCCACACACAAAGGTTCATGGGGAAAAACTCCTTGGTTAGACG
GCTTGATCTATAACGCCATGACAGCGACATTAACGAATGCCGATAGCATCAGCAACAGCCATTTCTTGAC
GCCATTACCGGCAGACACTATTCAACGTCCCGTTGAGCCTATCATGGAGGTGGCTTCGATCAGTCAGCC
TTGGCCGTCGTGGAATGGGCGTGTACCGGAATCCTACAGCGCTTTTTTTGCACGGGGAGCTCAACATCT
GTCCCACCGTAACCGCGCCTTAACCTGGGGCAATATGGCGACACTGTTAAAAGAGCGTTATGTCAGCAT
TTTTGATGTTAAGTACCCCGGCCATGATGAACTCACGCGAGTCCCCGCACTGGAGAAACAGCAACTGA
CCATCATTCCGGCTAACCAATACAACGACAGCGATGATCCTTTGCGTCCGGTGTTAAACCCTGCCCGCC
TGCAAGGAATGGCCGAATGGTTACAGCAAAAAGGCTCTCCATGGGCTGAGATTGAGATCAGCAATCCTC
AATACATTGATGTAAAAATCAATTACAACGTGATTTTTAAGCCGGATGTTAACGAAGATTTTGGTTATCGC
CAGCTACGACAGCAACTCAGCGAGACATATATGCCCTGGAGCGTCGATGAGAAACGCCCCGTCACGCTG
AATAACAGCATTAGTTATTACCAATTACTGGCAACTATCCAACAGCAGCCGCTGGTTGAGCGGGTCACT
TGTCTGACGCTGCGTCGGTCGGAAGCGGCTAAAGACAGTGATATCACCGCATCAGTGGAAGCCAAAGA
TAATGAAGTGCTGATTTTGGTCTGGGAAGCAGATGACAAGCTGCAATGCCGAGGAAATAACTATGAGTA
ATCAAGATGCACTATTTCACAGTGTGAAAGACGATATTCACTTTGATACCTTGCTGGAACAGGCTCATCA
GGTGGTTGAGCAACAGGCAGGAAAATTGTGGAGCGACACAGCGGAACACGATCCCGGTATCACCTTT
CTGGAAGGACTCAGTTACGGTGTGTCAGATTTAGCTTACCGTCATACTCTTCCTCCGACAGACTTACTG
ACCCCTGCCCCTAATGAGCAGAAACAGCAAGATGGAATTTTTCCCGCTGAATTTGGCCCCCACAATACA
CTGACTTGTGGGCCAATCACCACTGATGATTATCGGAAGGCATTACTTGATCTGCATAGCAGTGACTGG
ACAGGAGCAAAGCCAGAGTCAGAACAGGACAAAGGGGATTTTCTGTTCCGTAACGTGCAACTGGTAC
GTGAACCGGAAACACAACGCTATACCTATTGGTATGATGCCACCAAAAGGGAATATAGCTTTGTCGAGC
GTAAAGATGCGAAAAAGTTTACCCTGCGGGGAAATTATTGGCTTTATCTGGAACCGACCCGTCGTACTC
AGGAGAATTTGGATAATGCTACCCAACAATTAATCGATTTTTTGACTCAAAACCGCAATATTGGTGAATT

GGTCAGTCAGAGAATCTGGACAAAACCGGTTGATTTTCCGCTATTACTGGATATTGAACTGGATGACGAT
GTGCAAAATGTCGCTGGTATTTTTGCTGCCGTCTATAGCACAGCAGAACAGTATCTGATGCCTGAGGCGC
AGCGTTTCAGTACCGAAACACTACAAGATGCGGAAATGCGTAATGATGAAATCTTTGAAGGACCACGA
TTAGAACATGGCTGGATCCCTGAATTGCCGACAGCCCGTGATTATACCAAACGGCTCAAGCTCAATCTTA
GCCGGCTGGTCAATAAGCTGCTTGAGATTAAGGGAATACAGAATGTAAATCGTCTTCGACTGAATGAAA
ATTTTGATAAGACCCTGATTGAGCCGATTAAAGGGGATAGCTGGTCATGGTCAATAAAAGAAGGCTACTA
CCCTCGTTTGTGGGGTAAAGATCCGATTCATCAATTGGCACAACATGATGGCCCACTGAGGGTGATCGC
CAAAGGTGGGATCAGCGTTACGGTTGATGAAAACAGCATTCGAAAAAGTTTATCCTTATCCCATCCTCCT
CTTATTCAGAATAAACCTGTGCTATTGGCTTATGGTCGGCATCGCGATGTGGGTCGCTATTACCCCGTCAG
CGATACGTTGCCCGCTTGCTACAAATTACAGCAGCCTTTATCTGAAAGTGAACATGCGCAACGCCTGTTA
TCACTTCATCAATTTATGCTGCCATTTGAACAATTGTTGGCTTGCGGCTGCCAGCAAATTGCCATGCTGC
CAAAATTGTTGGCTTTTAAGCGTGAGGGATATGAAGTTTGGGGTGATCAATGGCCATTTAAGCCGGGTT
CTGTCAATGATGATGCCCACCAAAATTACGCTTACGAATTAAAGAAAGTACTAAAAGAGATTGCACACGA
TAACGATCACGAATTGGATATTGTCAATTATCTGCTCGGATACTTTGGCACTCAGCGAGCACCACGTACC
TTTACCACCAACATCAGTGATTTTCGTGCCGTGCAACAAGGCTATCTGGCTCAACAACCGACATTAACT
TATCACCGAGCCAATATTCGGATCGATCAAGTTTCGTCGCTACAAAAGCGCATTGCTGCCCGTATGGGGT
TAGGGGGTGAATTATTTAAACCAAAGCCTGACTTGAGCAAACTGCCTTTTTATCTGGTAGAACATCGGG
CATTGCTGCCAGTCAAACCCAACAGCCAGTTTGATGAAGAGCAGAAGCCAGATTCGGTGACAGAAGA
AAGGAACTATCAAAATGATCAATATTACTTGGTGATTAAGCAAGCCGGTATTGATGGAAAACTGATACAG
GGACAGGTAATTGATTTAGTTCTCTATGAAGGAGAACAGAACGAAAACCGATTTACCGTGCGTGGTCAA
ATGATCACTAAAACCACAGGAGATCAGTTTTGGCTGGACGTGAGCAACAGTACTCAACTGGAATACAA
TCTGGAACGGGTAATGACCGCCGCCAAAGGGAAAAAACTCGTCTGGCAAAATAGCCCGGTATGGATGG
AAGATATGAACTACCGGCTGGCCTATGATAGTGATCAATCCTCATTGCCTGAAAACCAGCGGCGCCTGA
CCCGCACAGCACAAACCCCCTTCCCGGCCCTGATTGCCGCAGGTAATGAAATTACCCTGACAAAACAG
TTGGGGGGAGTGGGTTTGGAGCAAGAATCTTCTAATGAATCAGAAAAATTATATGCAAAAGTTGTCAGC
TTTGATCGCATCAAGGGAACTTTGATTATTGAGCGTCAGAAACATTCTACACTGGCATTCCCTGCCGCAG
AAGAAGCCTGGCGGCACAGTTGGCATTTTTCGGGTAAGGAATATGAAAATACGGACCGTTTCTCATTTG
TGATTAGCGTGGTGGTAAATAGTGATTTAATTAAATCACGTGAGCTCAAAGTTGATCCATATCAATTGGAA
GAGTGGGTTAAGGAAACTATTCTCACCGAATTCCCTGCCCATATTTCCATGATTATTCACTGGATGGATCG

GGAGCCATTTTTAAACTTCGGCAATACGTATCAACGTTGGCAAAATAATGGCGCACCATTAGGGGATGCC
GCTTATTCGATTCTGGAAAGTTTGACGCTGGGTAAATTACCTTCCGCTTTCAAAGGAATTGGGTCAATGC
GTATTGCCACACCACATCAGAGAGAGGAAGTTGTTGGTAAGAATGGTGAACAATGGAATACCAATAGAA
TAAAACAAAACGAATTATTCTATATTCCGAAAGAGAAAGAATAAAAAACAATTAGCGACTAACCAACATT
TTAAATTAAAAATATTTTATGGAGTTAACAATGAAAGAAATTCGTTATTCTGCAAAAATACAAGAACAAC
AAACATTATCTGACGCAAAAGGTGTTGGACCTGATATTGATAAATTAAAAGATAAGTTTAAAGAAGGCAG
TATTCCCTTACAAACCGATTTCAATCAATTAATTGATATTGCCGATATTGGGCGTAAAGCGACTGGACAGG
CTCCCAATCAAAATGGCCCGGCAAAAGGAATGCGTTTGGGCGAAGATGGGCTTTTACAGTTAAAGTTAG
ATGAGAAATACGCTCATGCAAATTACAGTCCATTAAGATTGCATGATGATGCATTGGTTGTAGGAGTAGGC
AATGGTTTGATAAATTTTAAAAAATCAAATGAAATAGGTGTTAATGATGGAAATGGTATTAGGATAGATGAT
AATAAGGTTGCAGTAGAACTGGCTGATAATAAAGGTTTGGTTGTTGATAGCAATGGTATCGCCATAAAACC
AGGCAATGGGGTGAAGTTTAGTTCAGATGGTGCATTAACAGTAAATTCGGCTGATTCAACTATTAAAGTT
GATGGCTCAGGAATAAAAGTTGCTACAGGTTGGGGAGTTAAAGTTGGTGGTGAAGGTTTGGATGTAAA
TGTCCGCACGGATGGTGGTATTGGTAATGATTCGTCAGGTATTTATGTTAAAACAGGTAATGGTATTAAGTT
AGATAATGATCAAGTTGCAGTAGAACTAGCTGATAATAAAGGTTTAGTTGTTGATAGTAATGGTATCGCCA
TAAAACCCGGCGATGGAATGCAATTTAGTTCAGATGGTGCATTACAGTTAAAATTAAATAAGAATTATGCC
GATAGATTTTATAGCCCTTTAATATTGCGTAATGATGTATTGGCTGTAGGTATAAGCCATGGTTTGACAACTT
ATCAAGGATCATATGCAATAGGTGTTAATCCAGGAAATGGTCTTAGGATAAATGACGATAGTGTTGCAGT
GAAACTAGCTGATAATAAAGGCTTGGTTGTTGATAGCAATGGTATCTCCATAAAACCAGGCAATGGAATT
AAGGTAGATGCTGATGGTGTGGCTATCGATCCTAATACTGTATTGCCACGTGGTATGATTATGATGTTTTCT
GGTAATAGCGTACCAGAAGGTTGGGCTATTTGTGATGGCTATAATAATACACCAGATTTTCTAAATAGATCT
ATTGAGGGAAATACTAGGAATATTAAAAGTAAAAATGGTATAGATATGACTATACAATATACTGCAATATATAT
CATGAAATTATAATTAACATTAAACATGGCAATGAGCGTTTTAAAATACGCTCATTGACCAATTTTTGGCAA
GGTGGAATTTGCGGGTTTTATGATGACCAACACTGATAAATATGGTGTTAATCTTATTAATAGAATAACTAT
CTCCATTGATATGTTATATTTTAACGATGATAACATAATGGAGAACTGCTCATCTATATTTAATAGAAAACTAA
AAAATATCATCAATAAAAATATCAAAAACCTGACATTGAATGTAAATGGGAAGTTATCTCTTGAAAGGGTA
ACTCTGGATCTGGGAAATATTGAAATTGATTTATTTGAAAGGGAAATAACAAGAAGATTGGAAATAGAAT
TAAAAAAGAACTTAAGAAATCATTATGGTTACTCTCAATGTATTTTAAATAAAGAGAAAATATATCCACAC
CAATCAGAAAAAGAAAATAGATTTTTATTAATAAAAGAAATAGATCATTACTTACATAAGGGAAACTGGTT

ATCTGATCACAGAAAGCATCATATTACAAATACTGTGCATATTAATAATGAATTATGGCAACAGATTAACCA
CCAGCCACAACAATGGCTACCTATGTTAGCCAAACACTGCTTACAGCCTGCGGGGCTAAGTCGCTTAAT
TCAACTCTGCCAGCCAGACGTTCTTGCTATGCTTTGCCAGTTATTTACTGAAACAACAACATCCGAATTT
GTTAATTCGCAAGCGCAGGTAATTACAGATAAAAAAGTGAAACCAGAAAAATTGAGTTTGGCTGCGGAA
TATTATCTGCAACATCAGGTGAAATATCCCCATGCCACACAACAAGCACTTGCACAACAAACTCAAAATA
AGCAAACACCGACAATAAATATTATTCAGAATAATAACAGTCTATTAAAACAGACTTACAATGAAACTGAT
ATGCAGATGGATACAGTACTGCCATTTGCAACATACCATCAGCCAACAGCTACGCAACAGCATTATGGTC
TTATAAAAGAGGCGGTAAAACAAAGTCTTCCAACTTTACAAAACAGAGAGCAACATAAGGAATTACCG
AGATCAACTTCACCAGCGAAAATATCATCCATACAACCCAGTAATGGAATTCAGACTCCATCATCACAATC
CGTTTTAACCATCAGCAATGCCGGTTGCATGATTTTATGGCCGTTATTGCCCATATTTTTCCGTACCTTTGAT
TTACTGGAAAAGAATCAATTTATCAGCCTTGAAGCACAAAGAGAGGCGGTCTGTTTACTTGATTGGCTG
ATTTGGGAAGAAGAGGAAATACCTGCATGGCGATTGACTCTCAATAAAATACTTTGTGGATTACCCATAC
ATGACAACGCGCGATGGCGTACCCCAAAGCCAGAACAACAAATTGCCATCAACCAATGGCTGGAAAA
AATCATCGAACAACTTCCAGCCTGGAAAAAAATGGGAATAAACGATGTGCGACACTTATTTCTGCAACG
TTCCGGGGAATTAAGTGAGTTAAATGGAATAAAAAACATCCATATTAAACCCGAAGTTTATGATGCACTG
ATAAGCGAATGGGCGTGGCCAATGAATATCGCGAGCTTTAGCTGGTTGCAACACCCTATCACCATAACGT
GGTCATAACCATCAAACACAGTAAATTAGTCTGAGTAAAAATTTATGAATACAGCGCCTGTTTTCCCTGAT
TTATTTCAGCAACATAATGATCCTCATTTAATGTTCTTATATAGCCAACTGGAACGCATCGATCTCGTTCTG
CAATACCATTTCCATTGCATATCAGGCCAGAAAGGTGACCTCCTTGATGAGTTCTTGTTAAGCGAAGAAG
ATGTGATAAACAGACTGGATAACCCTCTGGGAAAACCTCATTGGCTCACCGATGATTGCCTGACAATAC
CGCACACCGTCACGCCCGTGGCATCCCGATTAACTGACCTGATTACACATTTTGAACTGACTGATTTTGA
AAGGGATGTTTTGTTGTTAGGTTTACTGCCCCATTTCGATAACCGCTACTATCACCTGTTTGCTCTGGTTC
AGGGAGAACAACAATCTCGGCTGCCCGCTTTTGCGTTGGCTTTGGAACTGTTTTGCCACTCAATGCTG
GAAAAACAGGCACAGCAGGCCAGTTTTTTACACCAGGCTCCCTTGGTTGGCTGCCAACTGTTATCTAT
TGATAGTCGTCAGAAAACGTTATCCTGGCTCCAGACACCCTTTATCACAGACAGCAGCGTATATCATTTT
TTACTTGGGCATCACTATATTGCACCTGCTCTGGAACATTGTGTTGAGTGGATAACGCCCACAGAAATCG
GCAATTATCCAGCAGGATTAAAACAAGCGTTGGGTAATGTACTGTTATCTGATCATGATGATATCCGGCCC
GTTATCTTACTGCGGGGCATGGCCGATAGTGCCAGATCCCATGTCGTTGCTAATATGATGGCATCAGAAC
AACGGCAAACGCTGCTTGTGGATATCGCCAGCTTAGCTGATAACGATGACAACTCCCTGTTTTGCCATAT

AAAAGCCATTTTGCGAGAAATTCGTATGCGTGGTGCCTGTTTATTGTTACGAAACTTTTGCTTATTTGCA

GAACAGAATAAACACTTGCTGGATCCCCTGTCAGCCTTATTAAACCAATCTGAATTAAGGGTTATCTGTC

TGGTTGAACCACACTCACCGTTAGTATGGCTGAAAAAGATATCGACATTACTGATCGAAATGCCCGCTTT

CACTTCGGTGGAAAAAGCAAGATTATTGACGGCCCATTTACCCAATCACTGTGCAAATGATATAGATGC

AATAACCTTAAGCCAACGTTACGCATTTAATCCTGAAACCCTGCCATTAATTTTACAAGAGGCGAAACTTT

ACCAGCAACAGCGAGATCCTTCAGGTATTTTGCAGCAATGTGATATCCATAAGGCACTCAATTTACGCGC

CCAGCAAAATTTTGGTCAATTGGCACAACGCATTACCCCTAAACGTTCATTAAAAGATTTATTGGTATCTG

ATGATATTGCTCAACAGATACAAGAAATACTCGCAGCCATTAAGTATCGGGAACACGTCCTGGCGGGAG

GTTTTAAAGATAAGATTGGTTATGGTACGGGTATCAGCGCCCTGTTTTATGGTGATTCTGGCACCGGAAA

AACTATGGCAGCAGAAGTGATTGCCAATTACATTGGCGTTGACTTAATCAAAGTGGATTTATCTACCGTC

GTGAATAAATACATCGGCGAAACAGAGAAAAACTTATCCCGTATCTTCGATTTGGCAGAACAGGATGCC

GGTGTTTTATTCTTTGATGAAGCGGATGCGTTATTTGGTAAACGCAGTGAAACCAAAGATGCGCAAGAC

CGACATGCCAATATTGAAGTCTCTTATCTACTGCAACGGCTGGAGAACTATCCGGGGTTGGTTATTTTATC

CACCAATAATCGTAGCCATTTGGACAACGCCTTTAATCGCCGTTTTACCTTTATTACCCGCTTTACTTACCC

GGATGAAACCATCCGCAAGAAAATGTGGCAGGCAATCTGGCCTGAAAATATAAAAGTCGCCCAGGATGT

GGATTTCGATAAATTAGCTCAACGAACAAGCGTAACAGGGGCGAATATTCGTAATATTGCTTTATTGTCTT

CACTGTTTGCCGCAGAGAATAACCATAATGAAGTCAGTAATAAAAATATTGAAACAGCATTATCACGAGA

ATTAGCCAAAGTAGGCCGTTTAATTTTTTAAAATTTATAGCAATGCAAATATCTAACAATTAAATGTACTGA

TAGGTTAATTGTTATTACCATTATAAATTAAGAGGATTTTTTATGTTAAAAACGCAAACCATTATTGATGTCA

ATAACGCAATGAAAACTATGCTGGAGAAATATCTGGATAATAAAATTGCAATTCGTTTTGACTTACCTGAA

TTAGATACCGTGCAATCTGATGCGATGGTGAGTGTCTTTCTGTATGATATCCACGAAGATCTACAGCTTCG

TTCGACAGAATCCAGAGTGTTTAATGCCACTTCAGGGAGATTATTGCCGGGTTGGGTCAATATTAAATGTA

ATTACCTGATTACCTATTGGGAATCGGCTCAACCAGCAACCGATTCCAACAGCTCAGAATGTCTACCGGA

TAATCAGGCAATAAAAGTCATGTCACAAGTCTTGAATGCTCTGATTAATAATCGCCAATTGGCAGGTATTC

CCGGTGCTTATACTCAGGTTGTCCCCCCGAAAGAAAGTTTAAACAGTTTAGGGAATTTTTGGCAATCAC

TGGGGAATCGCCCCCGACTTTCTTTAAATTACTCAGTGACAATACCGATTAGCCTCACCGATAAGCAGGA

TAAGGCGACGCCGGTAAGCACCATTTCTTCTACAGTGGAACAAATTGCATCACTCAGTCAGGAAAAGAT

TAGCCATACTTTGCGAGAATTATTGATTACGGAACTACAGATACAACCTGTTACAGAATCAGAAAGTGAA

GAGAATATCAGGCTGGCACTGAGCAAAGTTGAACTATTAACAATGAAAAAGGCTATAACTCCAGACAAT

TCAGCAGAAATTTCCATATCCTTGTCTGTATCAGGTATTACCCATCAGAAATATCTACAGGATATCAATAAA

ATTTTTGATCGCTGGTTAAATGACAACAAAATTGTTACTACAGTCGATAATTATGGAATCAGGATTAAGGAT

ATAGAAAAAAACCATCTTTTCGGCATTTAATTTATTGCTTTTCCATAGCTCATTAAATAACAGTTTATTTAAA

TCGGAGTATTATT;

The complete TcsT coding sequence (SEQ ID NO: 48):

GATGTTAGCTTTCGTCTGAGCGGGGCAACCAGCAGCAGCTATGGTGTTTTTATTTCTAATCTGCGTAAA
GCACTGCCGAATGAACGTAAACTGTATGATATTCCGCTGCTGCGTAGTAGCCTGCCGGGTAGTCAACG
TTATGCACTGATTCATCTGACCAATTATGCCGACGAAACCATCAGCGTGGCAATTGACGTTACCAATGT
ATATATTATGGGCTATCGTGCCGGTGATACAAGCTATTTTTTTAATGAAGCAAGCGCAACCGAAGCAGC
AAAATATGTTTTTAAAGACGCCATGCGTAAAGTGACCCTGCCGTATAGTGGTAACTACGAACGCCTGC
AAACAGCAGCAGGAAAAATTCGTGAAAATATCCCGCTGGGCCTGCCGGCACTGGATAGCGCAATCAC
CACCCTGTTTTATTATAACGCCAATTCTGCAGCAAGCGCCCTGATGGTTCTGATTCAGAGTACAAGCGA
AGCAGCACGTTACAAATTTATTGAACAGCAGATTGGGAAACGTGTCGACAAAACCTTTCTGCCGTCA
CTGGCAATTATTAGTCTGGAAAATAGCTGGTCTGCACTGAGCAAACAAATCCAAATTGCAAGCACCAA
TAACGGGCAGTTTGAAAGCCCGGTTGTTCTGATTAACGCCCAAAATCAGCGTGTGACCATCACCAATG
TGGACGCAGGTGTCGTTACCAGCAACATCGCCCTGCTGCTGAATCGCAATAATATGGCA;

The complete Xk-LysR sequence (SEQ ID NO: 49, the underlined portion is the lysR coding region): 
TTAGTCACTCAAGAGTAATTTGCCTGCTTTTAACATAATGATATCGCTTATCAGCCAAACCTAGCTCTAATA
ACATTTAAATTAATCTTATGAATAACAAGGTTTATTTATCATATTCCATGAACATTTTATTGTTATTTAATAAGG
TTTATTTATTTAAAAATCATATTAATTAATGTAATAATTCTTATATTCAATTGTTGACAAATATTAATGTCTATATA
TGATTATTGCACAATTAATAATTTCCACTGTGATGAATTACCACATCAAATCATGTAATTAAAATAAAAATGT
AACAAATTTATTAATAATTTCATAACACCAGGAATAAGTCCTATTGAATTTAAAAAGGAGTATAGGTGTTTA
TTTCAAAAGACTTAGCAAATTTTATTGTCGTAGCCAAAAATAAATCAATAAATAAGGCAACAAAGGAGCT
TTTTATTACACAGTCTCCAATTAGCCGAAGCTTAAAAAAATTGGAGTTCTCATTAGGTGTAAAACTCTTT
GTCAGAAAAAACCAAGGATTAGTTCTAACGGAAGAAGGGGAGTGTCTATACAATAAAGTGTTATCAATT
TATGAACAACTTAAAAATATCGAAAATGATTACAAGAATCATAAATCACCCTATTGTGAAATTTATAAAAA
ACAACATAATGACTTAACATAATAAAATAACAAGATAGATAAAAATATATTTCAGATAACTATTGAATCATAT
 
TCAATAGATTTCATTATATACATAAATTAAAAAGTAAATTTAACATGCCTAAATATCTTAGAGAAGCAGCAGA
AGAAAAGACTTCTGTACAAAAACATTCTACCGATAGCAATCTTCTCAACTCAGGGAACTAATATCGCGG
ATACCTAACAATACAATAATGCACTTGAAGAAAGTGCATTCAAGTTGAATTAAAATAAAACGTTGAAGGA
AAAGTATCACGAACCACCAGCTGGACAGGCAGAAATCCTTTTCCCCAGCAGAGCCACCTTCAAGGTT
CATGCGATCAAGCGAGATGGCAAAAATACCTATGTCTTGCTAAGTGATATTTCCTCTGATGCCAACATAGA
TACAGATATAAAAGATATATTTTCAGGAAAAAAGAT;
BlaM (β-lactamase) gene sequence (SEQ ID NO: 50): 
ATGAGTATTCAACATTTCCGTGTCGCCCTTATTCCCTTTTTTGCGGCATTTTGCCTTCCTGTTTTTGCTCAC
CCAGAAACGCTGGTGAAAGTAAAAGATGCTGAAGATCAGTTGGGTGCACGAGTGGGTTACATCGAACT
GGATCTCAACAGCGGTAAGATCCTTGAGAGTTTTCGCCCCGAAGAACGTTTCCCAATGATGAGCACTTT
TAAAGTTCTGCTATGTGGCGCGGTATTATCCCGTGTTGACGCCGGGCAAGAGCAACTCGGTCGCCGCATA
CACTATTCTCAGAATGACTTGGTTGAGTACTCACCAGTCACAGAAAAGCATCTTACGGATGGCATGACA
GTAAGAGAATTATGCAGTGCTGCCATAACCATGAGTGATAACACTGCGGCCAACTTACTTCTGACAACGA
TCGGAGGACCGAAGGAGCTAACCGCTTTTTTGCACAACATGGGGGATCATGTAACTCGCCTTGATCGTT
GGGAACCGGAGCTGAATGAAGCCATACCAAACGACGAGCGTGACACCACGATGCCTGTAGCAATGGCA
ACAACGTTGCGCAAACTATTAACTGGCGAACTACTTACTCTAGCTTCCCGGCAACAATTAATAGACTGGA
TGGAGGCGGATAAAGTTGCAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGGCCAGATGGTAAGCCCTCCC
GTATCGTAGTTATCTACACGACGGGGAGTCAGGCAACTATGGATGAACGAAATAGACAGATCGCTGAGAT
AGGTGCCTCACTGATTAAGCATTGGTAA;
Cre gene sequence (SEQ ID NO: 51): 
ATGTCCAATTTACTGACCGTACACCAAAATTTGCCTGCATTACCGGTCGATGCAACGAGTGATGAGGTTC
GCAAGAACCTGATGGACATGTTCAGGGATCGCCAGGCGTTTTCTGAGCATACCTGGAAAATGCTTCAGT
CCGTTTGCCGGTCGTGGGCGGCATGGTGCAAGTTGAATAACCGGAAATGGTTTCCCGCAGAACCTGAAG
ATGTTCGCGATTATCTTCTATATCTTCAGGCGCGCGGTCTGGCAGTAAAAACTATCCAGCAACATTTGGGC
CAGCTAAACATGCTTCATCGTCGGTCCGGGCTGCCACGACCAAGTGACAGCAATGCTGTTTCACTGGTT
ATGCGGCGGATCCGAAAAGAAAACGTTGATGCCGGTGAACGTGCAAAACAGGCTCTAGCGTTCGAACG
CACTGATTTCGACCAGGTTCGTTCACTCATGGAAAATAGCGATCGCTGCCAGGATATACGTAATCTGGCA
 
TTTCTGGGGATTGCTTATAACACCCTGTTACGTATAGCCGAAATTGCCAGGATCAGGGTTAAAGATATCTC
ACGTACTGACGGTGGGAGAATGTTAATCCATATTGGCAGAACGAAAACGCTGGTTAGCACCGCTGGTGT
AGAGAAGGCACTTAGCCTGGGGGTAACTAAACTGGTCGAGCGATGGATTTCCGTCTCTGGTGTAGCTGA
TGATCCGAATAACTACCTGTTTTGCCGGGTCAGAAAAAATGGTGTTGCCGCGCCATCTGCCACCAGCCA
GCTATCAACTCGCGCCCTGGAAGGGATTTTTGAAGCAACTCATCGATTGATTTACGGCGCTAAGGATGAC
TCTGGTCAGAGATACCTGGCCTGGTCTGGACACAGTGCCCGTGTCGGAGCCGCGCGAGATATGGCCCGC
GCTGGAGTTTCAATACCGGAGATCATGCAAGCTGGTGGCTGGACCAATGTAAATATTGTCATGAACTATA
TCCGTAACCTGGATAGTGAAACAGGGGCAATGGTGCGCCTGCTGGAAGATGGCGATTAA;
Rluc (Renilla luciferase) gene sequence (SEQ ID NO: 52): 
ATGACCAGCAAAGTTTATGATCCAGAACAGCGCAAACGTATGATCACCGGCCCGCAGTGGTGGGCACGT
TGCAAACAGATGAACGTTCTGGATAGCTTTATCAATTATTATGATAGCGAAAAACATGCCGAAAATGCAG
TTATTTTTCTGCATGGTAATGCCGCCAGTTCATATCTGTGGCGTCATGTTGTTCCACATATTGAACCGGTG
GCACGTTGCATCATTCCGGATCTGATTGGTATGGGTAAAAGCGGTAAAAGCGGCAATGGTAGTTATCGTC
TGCTGGATCATTATAAATATCTGACCGCGTGGTTTGAACTGCTGAATCTGCCGAAAAAGATCATTTTTGTT
GGTCATGATTGGGGTGCATGTCTGGCATTTCATTATAGCTATGAACATCAGGATAAAATCAAAGCAATCGT
TCATGCGGAAAGCGTTGTAGATGTCATTGAAAGTTGGGATGAATGGCCGGATATTGAAGAAGATATTGCA
CTGATTAAAAGCGAAGAAGGTGAAAAAATGGTTCTGGAAAATAATTTTTTTGTTGAAACCATGCTGCCG
AGCAAAATTATGCGTAAACTGGAACCGGAAGAATTTGCAGCATATCTGGAACCGTTTAAAGAAAAAGGT
GAAGTTCGTCGTCCGACCCTGAGCTGGCCGCGTGAAATTCCGCTGGTTAAAGGTGGTAAACCGGATGTT
GTTCAGATTGTTCGTAATTATAATGCATATCTGCGTGCAAGCGATGATCTGCCGAAAATGTTTATTGAAAG
CGATCCGGGTTTTTTTAGCAATGCAATTGTTGAAGGTGCAAAAAAATTTCCGAATACCGAATTTGTTAAA
GTTAAAGGTCTGCATTTTAGCCAGGAAGATGCACCGGATGAAATGGGTAAATATATTAAAAGTTTTGTTG
AACGTGTTCTGAAAAATGAACAGTAA;
Xk1N30 (SEQ ID NO: 54):
   VYSLKQKEEKKNHSPRPTQNTVNGDHAITP;
Xk2N30 (SEQ ID NO: 55):
   MIYGYDNKMVYRKRQESNTALEALHLDIPD;
Xk3N30 (SEQ ID NO: 56):
   MPVYENKEKRKYSKNPLHNTSTINQFTVQD;
Xk1N50 (SEQ ID NO: 57):
   VYSLKQKEEKKNHSPRPTQNTVNGDHAITPRIAELRKNFQQLNSQTVRPR;
Xk2N50 (SEQ ID NO: 58):
   MIYGYDNKMVYRKRQESNTALEALHLDIPDTAEVSAAYQRDLSSVMTRFQ;
Xk3N50 (SEQ ID NO: 59):
   MPVYENKEKRKYSKNPLHNTSTINQFTVQDLDSASQQETATPLLTNEFKQ;
Xk1N70 (SEQ ID NO: 60):
   VYSLKQKEEKKNHSPRPTQNTVNGDHAITPRIAELRKNFQQLNSQTVRPRVAPRPLHSLSKTGPFQQATK;
Xk2N70 (SEQ ID NO: 61):
   MIYGYDNKMVYRKRQESNTALEALHLDIPDTAEVSAAYQRDLSSVMTRFQNKTDSILEESKQPSPLKQKP ;
Xk3N70 (SEQ ID NO: 62):
   MPVYENKEKRKYSKNPLHNTSTINQFTVQDLDSASQQETATPLLTNEFKQGHYVNHSKLPAGTKLSEIAM ;
Xk1N90 (SEQ ID NO: 63): 
VYSLKQKEEKKNHSPRPTQNTVNGDHAITPRIAELRKNFQQLNSQTVRPRVAPRPLHSLSKTGPFQQATK

APIASEIITPVSAATKTFTS;
Xk2N90 (SEQ ID NO: 64): 
MIYGYDNKMVYRKRQESNTALEALHLDIPDTAEVSAAYQRDLSSVMTRFQNKTDSILEESKQPSPLKQKP
SEKKKYAKKALKNFAAHAGY;
Xk3N90 (SEQ ID NO: 65): 
MPVYENKEKRKYSKNPLHNTSTINQFTVQDLDSASQQETATPLLTNEFKQGHYVNHSKLPAGTKLSEIAM

LGSHDAGTYAYSRRKSGFAS;
Xk1N110 (SEQ ID NO: 66): 
VYSLKQKEEKKNHSPRPTQNTVNGDHAITPRIAELRKNFQQLNSQTVRPRVAPRPLHSLSKTGPFQQATK

APIASEIITPVSAATKTFTSNTQNPKVTPTSTSAPTNGVK;
Xk2N110 (SEQ ID NO: 67): 
MIYGYDNKMVYRKRQESNTALEALHLDIPDTAEVSAAYQRDLSSVMTRFQNKTDSILEESKQPSPLKQKP

SEKKKYAKKALKNFAAHAGYSHINSYQDEFVNFKDNNYNL;
Xk3N110 (SEQ ID NO: 68): 
MPVYENKEKRKYSKNPLHNTSTINQFTVQDLDSASQQETATPLLTNEFKQGHYVNHSKLPAGTKLSEIAM

LGSHDAGTYAYSRRKSGFASSLGSLLPAAFKTQNRTLRQQ;
M1 peptide fragment (SEQ ID NO: 69):
   YCGTNYNGCY.

The above description is merely preferred examples of this application, and is not intended to limit the application in any other way. Any person skilled in the art may make changes or modifications to the above-disclosed technical content to create equivalent examples. However, any simple modifications, equivalent changes, and alterations made to the above examples based on the technical essence of this application, without departing from the scope of the technical solutions of this application, shall still fall within the protection scope of the technical solutions of this application.

## Claims

1. A protein delivery system comprising a protein complex and a signal peptide, wherein
the protein complex enables the protein delivery system to target the cells of interest, the signal peptide guides the loading of effector proteins into the protein complex, and the protein delivery system is an extracellular contractile injection system from *Xenorhabdus khoisanae,* optionally *Xenorhabdus khoisanae* is MCB strain.

2. The protein delivery system according to claim 1, wherein the protein complex comprises a structural protein and a regulatory factor, and the structural protein is encoded by the *Cis* gene cluster of *Xenorhabdus khoisanae,* optionally the structural protein is encoded by the XkCis1-16 *Cis* gene cluster of *Xenorhabdus khoisanae* MCB strain, optionally the targeting recognition region in the *Cis* gene cluster is modified to recognize cells of interest.

3. The protein delivery system according to claim 2, wherein the targeting recognition region comprises the C-terminal amino acids 50-200 aa, 60-180 aa, 70-160 aa, 80-140 aa, 60-120 aa, 60-100 aa, or 60-80 aa region of the XkCis13 protein; optionally the targeting recognition region comprises the region of amino acids 355-419 in SEQ ID NO: 29; and optionally the targeting recognition region comprises the region of amino acids 360-406 in SEQ ID NO: 29.

4. The protein delivery system according to claim 2 or 3, wherein the targeting recognition region comprises a nucleotide sequence encoding a receptor, ligand, antibody, or a binding domain fragment thereof that recognizes a surface molecule of the cell of interest.

5. The protein delivery system according to any one of claims 2-4, wherein the XkCis1-16 *Cis* gene cluster comprises a nucleotide sequence consisting of the sequences set forth in SEQ ID NO: 47 and SEQ ID NO: 53 in the order from 5' end to 3' end, or a nucleotide sequence having at least 70%, 75%, 80%, 85%, 90%, 95%, or 99% or higher sequence identity with the nucleotide sequence consisting of the sequences set forth in SEQ ID NO: 47 and SEQ ID NO: 53 in the order from 5' end to 3' end.

6. The protein delivery system according to any one of claims 2-5, wherein the regulatory factor comprises the amino acid sequence set forth in SEQ ID NO: 34, or comprises an amino acid sequence having at least 70%, 75%, 80%, 85%, 86%, 87%, 88%, 89%, 90%, 91%, 92%, 93%, 94%, 95%, 96%, 97%, 98%, or 99% identity with SEQ ID NO: 34.

7. The protein delivery system according to any one of claims 1-6, wherein the signal peptide comprises the amino acid sequence set forth in any one of SEQ ID NOs: 44-46, 54-68, or comprises an amino acid sequence having at least 70%, 75%, 80%, 85%, 86%, 87%, 88%, 89%, 90%, 91%, 92%, 93%, 94%, 95%, 96%, 97%, 98%, or 99% identity with the above amino acid sequence;
optionally the signal peptide comprises the amino acid sequence set forth in any one of SEQ ID NOs: 60-62;
optionally the signal peptide comprises one or more of: the amino acid sequence set forth in SEQ ID NO: 38, 39 or 40, or a fragment thereof;
optionally the fragment is a C-terminal truncated fragment of the amino acid sequence set forth in SEQ ID NO: 38, 39 or 40.

8. The protein delivery system according to any one of claims 1-7, wherein the C-terminus of the signal peptide is connected to the N-terminus of the effector protein; optionally a linker sequence is interposed between the signal peptide and the effector protein.

9. The protein delivery system according to any one of claims 1-8, wherein the effector protein comprises any target protein to be administered to a subject or cells of interest;
optionally the effector protein comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cellular metabolism, gene-editing protein, antigen or immunogen, and/or pharmaceutically active protein.

10. An isolated polynucleotide encoding the protein delivery system according to any one of claims 1-9.

11. The polynucleotide according to claim 10, wherein the polynucleotide comprises any one or more selected from the group consisting of:
i) nucleotides encoding a structural protein in the protein delivery system;
ii) nucleotides encoding a regulatory factor in the protein delivery system; and
iii) nucleotides encoding a signal peptide in the protein delivery system; and
optionally nucleotides encoding an effector protein.

12. An expression vector comprising the polynucleotide according to claim 10 or 11.

13. A recombinant host cell comprising the protein delivery system according to any one of claims 1-9, the polynucleotide according to claim 10 or 11, or the expression vector according to claim 12.

14. A method for preparing the protein delivery system according to any one of claims 1-9, comprising culturing the host cell according to claim 13 to prepare the protein delivery system according to any one of claims 1-9.

15. Use of the protein delivery system of any one of claims 1-9, the polynucleotide of claim 10 or 11, the expression vector of claim 12, or the recombinant host cell of claim 13 in the preparation of a medicament, reagent, and/or kit for delivering a biological macromolecule.

16. The use according to claim 15, wherein the biological macromolecule comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cell metabolism, gene-editing protein, antigen or immunogen, and/or pharmaceutically active protein.

17. A pharmaceutical composition comprising: the protein delivery system according to any one of claims 1-9, and
optionally a pharmaceutically acceptable carrier or excipient;
optionally the effector protein comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cellular metabolism, gene-editing protein, antigen or immunogen, and/or pharmaceutically active protein.

18. A kit for delivering an effector protein, comprising: the protein delivery system according to any one of claims 1-9, or the pharmaceutical composition according to claim 17;
wherein the effector protein comprises a virulence factor or reporter protein derived from *Xenorhabdus khoisanae* MCB strain, a cellular or bacterial virulence protein, transcription factor, regulatory protein, hormone or hormone-regulating molecule, structural protein, transport protein, antimicrobial peptide, enzyme involved in cellular metabolism, gene-editing protein, antigen or immunogen, and/or pharmaceutically active protein.
